(19) Europäisches Patentamt
European Patent Office
Office européen des brevets

(11) EP 4 696 328 A1

(12) EUROPEAN PATENT APPLICATION
published in accordance with Art. 153(4) EPC

(43) Date of publication:
18.02.2026 Bulletin 2026/08

(21) Application number: 24784944.1

(22) Date of filing: 04.04.2024

(51) International Patent Classification (IPC):
*A61K 47/68* (2017.01) *A61K 31/4745* (2006.01)
*A61K 39/395* (2006.01) *A61P 11/00* (2006.01)
*A61P 13/10* (2006.01) *A61P 15/00* (2006.01)
*A61P 35/00* (2006.01) *A61P 35/02* (2006.01)
*C07K 16/18* (2006.01) *C07K 16/30* (2006.01)
*C12N 15/13* (2006.01)

(52) Cooperative Patent Classification (CPC):
**A61K 31/4745; A61K 39/395; A61K 47/68; A61P 11/00; A61P 13/10; A61P 15/00; A61P 35/00; A61P 35/02; C07K 16/00; C07K 16/18; C07K 16/30**

(86) International application number:
**PCT/JP2024/013882**

(87) International publication number:
**WO 2024/210162 (10.10.2024 Gazette 2024/41)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **05.04.2023 JP 2023061340**
**13.12.2023 JP 2023210652**

(71) Applicant: **Daiichi Sankyo Company, Limited**
**Tokyo 103-8426 (JP)**

(72) Inventors:
- **TAKAHASHI, Kazuki**
**Tokyo 103-8426 (JP)**
- **NAGASE, Akiko**
**Tokyo 103-8426 (JP)**

(74) Representative: **Marks & Clerk LLP**
**15 Fetter Lane**
**London EC4A 1BW (GB)**

Remarks:
The complete document including Reference Table(s) and the Sequence Listing(s) can be downloaded from the EPO website

# (54) THERAPEUTIC METHOD FOR LOW-DRUG-SENSITIVITY CANCERS BY ADMINISTRATION OF ANTI-MUC1 ANTIBODY-DRUG CONJUGATE

(57) Provided are a treatment agent for a cancer having low sensitivity to an existing anti-cancer agent, the treatment agent comprising an anti-MUC1 antibody-drug conjugate as an active ingredient, and a method for treating the cancer.

EP 4 696 328 A1

[Figure 1]

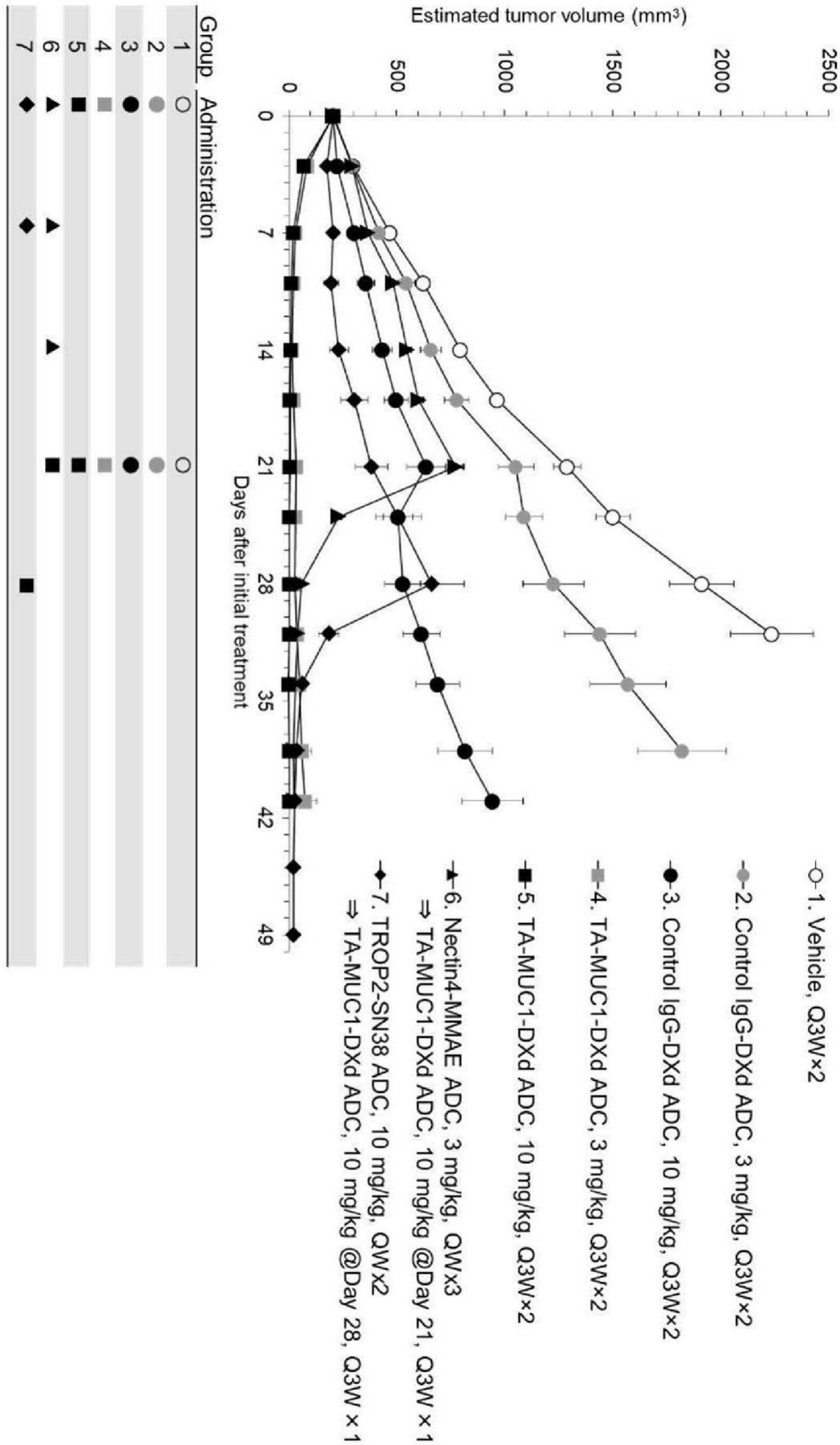
Estimated tumor volume (mm3)
0
500
1000
1500
2000
2500
0
7
14
21
28
35
42
49
Days after initial treatment
1. Vehicle, Q3W×2
2. Control IgG-DXd ADC, 3 mg/kg, Q3W×2
3. Control IgG-DXd ADC, 10 mg/kg, Q3W×2
4. TA-MUC1-DXd ADC, 3 mg/kg, Q3W×2
5. TA-MUC1-DXd ADC, 10 mg/kg, Q3W×2
6. Nectin4-MMAE ADC, 3 mg/kg, QWx3
⇒ TA-MUC1-DXd ADC, 10 mg/kg @Day 21, Q3W × 1
7. TROP2-SN38 ADC, 10 mg/kg, QWx2
⇒ TA-MUC1-DXd ADC, 10 mg/kg @Day 28, Q3W × 1
Group
Administration
1
2
3
4
5
6
7

## Description

### Technical Field

The present invention relates to a treatment agent and a treatment method for a cancer having low sensitivity to an existing anti-cancer agent, comprising administering an anti-MUC1 antibody-drug conjugate.

### Background Art

An antibody-drug conjugate (ADC) is a modality that can be expected to have a wider safety margin and have stronger drug efficacy than those of conventional anti-cancer agents, by delivering the drug in a cancer-specific manner. For example, an anti-Nectin-4 antibody-drug conjugate enfortumab vedotin (INN: enfortumab vedotin, PADCEV(R)) and an anti-TROP2 antibody-drug conjugate sacituzumab govitecan (INN: sacituzumab govitecan, TRODELVY(R)) have been approved as standard treatments for urothelial cancer, and an anti-HER2 antibody-drug conjugate trastuzumab deruxtecan (INN: trastuzumab deruxtecan, ENHERTU(R)) and TRODELVY(R) have been approved as standard treatments for breast cancer (Patent Literature 1 to 3). However, many cases do not respond to standard chemotherapeutics including these ADCs or become unresponsive to these chemotherapeutics by acquiring resistance after temporarily responding thereto. Since subsequent treatment options are limited, novel treatment methods are required.

Human mucin-1 (MUC1) is a highly glycosylated protein that is expressed on the apical membrane side of the epithelial lining in human normal tissues and forms the mucosal barrier. However, MUC1 in tumor tissues loses its expression polarity and becomes expressed in the whole cell membrane or even in the cytoplasm (Non Patent Literature 1). The overexpression of MUC1 is further found in various clinical cancer types (Non Patent Literature 1) and has been reported to lead to poor prognosis in patients with high expression compared with patients with low expression in the case of bladder cancer or the like (Non Patent Literature 2). In tumors, MUC1 with a new epitope exposed, called tumor-associated MUC1 (TA-MUC1), which exhibits a low degree of glycosylation, becomes expressed by a change in expression of sialyltransferase involved in glycan modification (Non Patent Literature 1).

An ADC having a drug with cytotoxicity conjugated to an antibody, which recognizes TA-MUC1 expressed on the cancer cell surface and exhibits internalization activity, can deliver the drug selectively to cancer cells and is thus expected to cause accumulation of the drug within cancer cells and to kill the cancer cells. The extracellular domain of MUC1 contains about 25 to 120 tandem repeat sequences each composed of 20 amino acids (Non Patent Literature 3 and 4), and a plurality of antibodies are capable of binding to one molecule of MUC1. In other words, compared with ADCs in general, such an ADC delivers more drug and is thus expected to exhibit stronger drug efficacy on tumors that did not respond to previous standard treatments.

An antibody-drug conjugate having an anti-MUC1 antibody and a topoisomerase 1 inhibitor exatecan as components is known as an anti-MUC1 antibody-drug conjugate (Patent Literature 4).

### Citation List

#### Patent Literature

Patent Literature 1: International Publication No. WO 2010/093395
Patent Literature 2: U.S. Patent No. 7999083
Patent Literature 3: International Publication No. WO 2015/115091
Patent Literature 4: International Publication No. WO 2019/219891

#### Non Patent Literature

Non Patent Literature 1: Nath S, Mukherjee P., Trends Mol Med. 2014; 20 (6): 332-42
Non Patent Literature 2: Qing L, Li Q, Yang Y, et al., BMC Urol. 2022; 22 (1): 114
Non Patent Literature 3: Gendler SJ, Lancaster CA, Taylor-Papadimitriou J, et al., J Biol Chem. 1990; 265 (25): 15286-93
Non Patent Literature 4: Hanisch FG, Muller S. Glycobiology. 2000; 10 (5): 439-49

### Summary of Invention

Technical Problem

An object of the present invention is to provide a treatment agent and a treatment method for a tumor that does not respond to an existing anti-cancer agent.

Solution to Problem

The present inventors have found that an anti-MUC1 antibody-drug conjugate exhibits an excellent antitumor effect on tumors that do not respond to an existing anti-cancer agent.

Specifically, the present invention relates to the following.

[1] A treatment agent for a cancer having low sensitivity to an existing anti-cancer agent, comprising an anti-MUC1 antibody-drug conjugate as an active ingredient.

[2] The treatment agent according to [1], wherein the anti-cancer agent is an alkylating agent, a platinum-containing drug, an antifolate, a pyridine metabolism inhibitor, a purine metabolism inhibitor, a ribonucleotide reductase inhibitor, a nucleotide analog, a topoisomerase inhibitor, a microtubule polymerization inhibitor, a microtubule depolymerization inhibitor, an antitumor antibiotic, an anti-hormonal agent, an EGFR inhibitor, a BTK inhibitor, a BCR-ABL inhibitor, an ALK inhibitor, a HER2 inhibitor, an angiogenesis inhibitor, a PARP inhibitor, an mTOR inhibitor, a membrane differentiation antigen targeting drug, or an antibody-drug conjugate.

[3] The treatment agent according to [1], wherein the anti-cancer agent is an antibody-drug conjugate.

[4] The treatment agent according to [3], wherein the antibody-drug conjugate is an anti-TROP2 antibody-drug conjugate, an anti-Nectin-4 antibody-drug conjugate, or an anti-HER2 antibody-drug conjugate.

[5] The treatment agent according to [4], wherein the antibody-drug conjugate is enfortumab vedotin, sacituzumab govitecan, datopotamab deruxtecan, trastuzumab emtansine, or trastuzumab deruxtecan.

[6] The treatment agent according to any one of [1] to [5], wherein the cancer expresses TA-MUC1.

[7] The treatment agent according to [6], wherein the cancer is selected from the group consisting of ovarian cancer, breast cancer, pancreatic cancer, lung cancer, colon cancer, gastric cancer, liver cancer, kidney cancer, blood cancer, endometrial cancer, thyroid cancer, leukemia, seminoma, melanoma, carcinoma, teratoma, lymphoma, sarcoma, mesothelioma, neuroblastoma, glioma, rectal cancer, adrenal cancer, skin cancer, brain cancer, uterine cervix cancer, gut cancer, intestinal cancer, head-and-neck cancer, alimentary canal cancer, lymph node cancer, esophageal cancer, colorectal cancer, ear, nose and throat (ENT) cancer, prostate cancer, bladder cancer, uterine cancer, biliary tract cancer, and metastases thereof.

[8] The treatment agent according to [6], wherein the cancer is breast cancer, lung cancer, or bladder cancer.

[9] The treatment agent according to any one of [1] to [8], wherein the anti-MUC1 antibody is an anti-TA-MUC1 antibody.

[10] The treatment agent according to any one of [1] to [9], wherein the anti-MUC1 antibody-drug conjugate is an anti-MUC1 antibody-drug conjugate, in which a drug-linker represented by the following formula:

[Chem. 1]

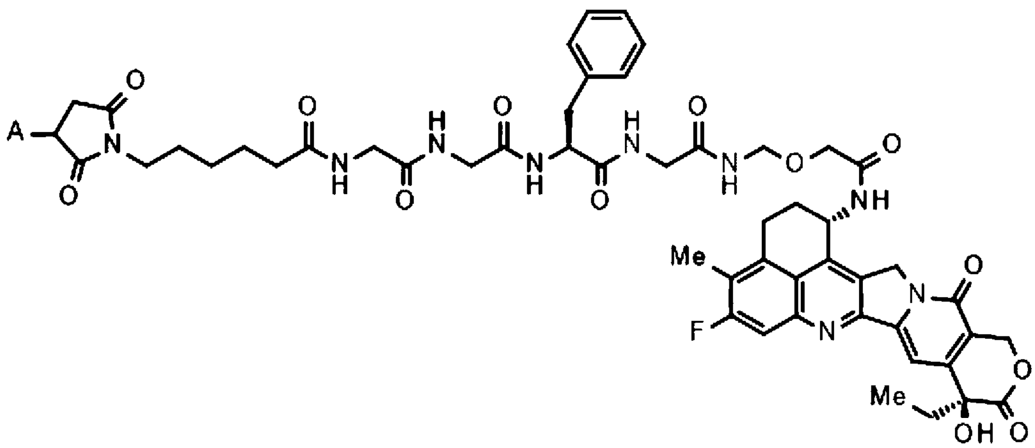

wherein A represents the connecting position to an anti-MUC1 antibody,
is conjugated to the anti-MUC1 antibody via a thioether bond.

[11] The treatment agent according to any one of [1] to [10], wherein the anti-MUC1 antibody is an antibody comprising a heavy chain comprising CDRH1 consisting of the amino acid sequence represented by SEQ ID NO: 1, CDRH2 consisting of the amino acid sequence represented by SEQ ID NO: 2 or SEQ ID NO: 7, and CDRH3 consisting of the amino acid sequence represented by SEQ ID NO: 3, and a light chain comprising CDRL1 consisting of the amino acid sequence represented by SEQ ID NO: 4, CDRL2 consisting of the amino acid sequence represented by SEQ ID NO: 5, and CDRL3 consisting of the amino acid sequence represented by SEQ ID NO: 6.

[12] The treatment agent according to any one of [1] to [10], wherein the anti-MUC1 antibody is an antibody comprising a heavy chain comprising CDRH1 consisting of the amino acid sequence represented by SEQ ID NO: 1, CDRH2 consisting of the amino acid sequence represented by SEQ ID NO: 2, and CDRH3 consisting of the amino acid sequence represented by SEQ ID NO: 3, and a light chain comprising CDRL1 consisting of the amino acid sequence represented by SEQ ID NO: 4, CDRL2 consisting of the amino acid sequence represented by SEQ ID NO: 5, and CDRL3 consisting of the amino acid sequence represented by SEQ ID NO: 6.

[13] The treatment agent according to any one of [1] to [10], wherein the anti-MUC1 antibody is an antibody comprising a heavy chain comprising a heavy chain variable region consisting of the amino acid sequence represented by SEQ ID NO: 8 or SEQ ID NO: 10, and a light chain comprising a light chain variable region consisting of the amino acid sequence represented by SEQ ID NO: 9.

[14] The treatment agent according to any one of [1] to [10], wherein the anti-MUC1 antibody is an antibody comprising a heavy chain comprising a heavy chain variable region consisting of the amino acid sequence represented by SEQ ID NO: 8, and a light chain comprising a light chain variable region consisting of the amino acid sequence represented by SEQ ID NO: 9.

[15] The treatment agent according to any one of [1] to [10], wherein the anti-MUC1 antibody is an antibody comprising a heavy chain consisting of the amino acid sequence represented by SEQ ID NO: 11 or SEQ ID NO: 13, and a light chain consisting of the amino acid sequence represented by SEQ ID NO: 12.

[16] The treatment agent according to any one of [1] to [10], wherein the anti-MUC1 antibody is an antibody comprising a heavy chain consisting of the amino acid sequence represented by SEQ ID NO: 11, and a light chain consisting of the amino acid sequence represented by SEQ ID NO: 12.

[17] The treatment agent according to [15] or [16], wherein a lysine residue at the carboxyl terminus of the heavy chain of the anti-MUC1 antibody is deleted.

[18] The treatment agent according to any one of [1] to [17], wherein the average number of units of the drug-linker conjugated per antibody molecule in the anti-MUC1 antibody-drug conjugate is in the range of from 7 to 8.

[19] The treatment agent according to any one of [1] to [17], wherein the average number of units of the drug-linker conjugated per antibody molecule in the anti-MUC1 antibody-drug conjugate is in the range of from 7.5 to 8.

[20] A method for treating a cancer having low sensitivity to an existing anti-cancer agent, comprising administering an anti-MUC1 antibody-drug conjugate.

[21] The treatment method according to [20], wherein the anti-cancer agent is an alkylating agent, a platinum-containing drug, an antifolate, a pyridine metabolism inhibitor, a purine metabolism inhibitor, a ribonucleotide reductase inhibitor, a nucleotide analog, a topoisomerase inhibitor, a microtubule polymerization inhibitor, a microtubule depolymerization inhibitor, an antitumor antibiotic, an anti-hormonal agent, an EGFR inhibitor, a BTK inhibitor, a BCR-ABL inhibitor, an ALK inhibitor, a HER2 inhibitor, an angiogenesis inhibitor, a PARP inhibitor, an mTOR inhibitor, a membrane differentiation antigen targeting drug, or an antibody-drug conjugate.

[22] The treatment method according to [20], wherein the anti-cancer agent is an antibody-drug conjugate.

[23] The treatment method according to [22], wherein the antibody-drug conjugate is an anti-TROP2 antibody-drug conjugate, an anti-Nectin-4 antibody-drug conjugate, or an anti-HER2 antibody-drug conjugate.

[24] The treatment method according to [22], wherein the antibody-drug conjugate is enfortumab vedotin, sacituzumab govitecan, datopotamab deruxtecan, trastuzumab emtansine, or trastuzumab deruxtecan.

[25] The treatment method according to any one of [20] to [24], wherein the cancer expresses TA-MUC1.

[26] The treatment method according to [25], wherein the cancer is selected from the group consisting of ovarian cancer, breast cancer, pancreatic cancer, lung cancer, colon cancer, gastric cancer, liver cancer, kidney cancer, blood cancer, endometrial cancer, thyroid cancer, leukemia, seminoma, melanoma, carcinoma, teratoma, lymphoma, sarcoma, mesothelioma, neuroblastoma, glioma, rectal cancer, adrenal cancer, skin cancer, brain cancer, uterine cervix cancer, gut cancer, intestinal cancer, head-and-neck cancer, alimentary canal cancer, lymph node cancer, esophageal cancer, colorectal cancer, ear, nose and throat (ENT) cancer, prostate cancer, bladder cancer, uterine cancer, biliary tract cancer, and metastases thereof.

[27] The treatment method according to [25], wherein the cancer is breast cancer, lung cancer, or bladder cancer.

[28] The treatment method according to any one of [20] to [27], wherein the anti-MUC1 antibody is an anti-TA-MUC1 antibody.

[29] The treatment method according to any one of [20] to [28], wherein the anti-MUC1 antibody-drug conjugate is an anti-MUC1 antibody-drug conjugate, in which a drug-linker represented by the following formula:

[Chem. 2]

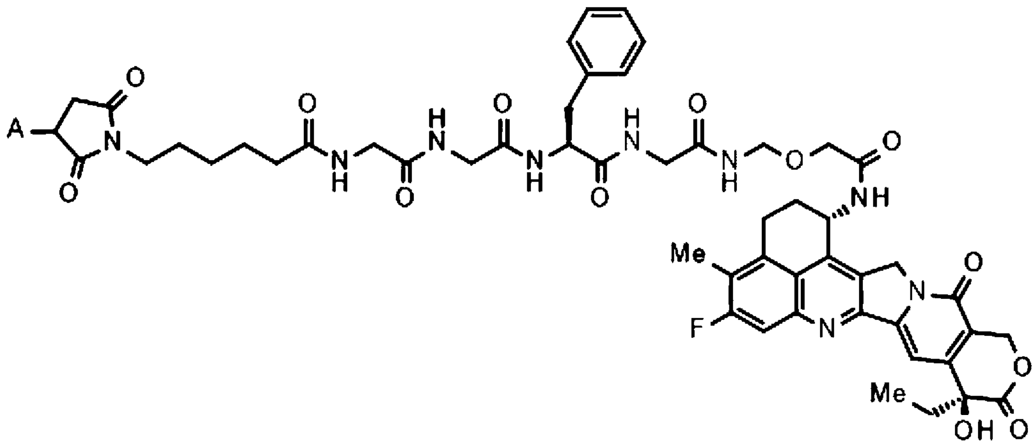

wherein A represents the connecting position to an anti-MUC1 antibody,
is conjugated to the anti-MUC1 antibody via a thioether bond.

[30] The treatment method according to any one of [20] to [29], wherein the anti-MUC1 antibody is an antibody comprising a heavy chain comprising CDRH1 consisting of the amino acid sequence represented by SEQ ID NO: 1, CDRH2 consisting of the amino acid sequence represented by SEQ ID NO: 2 or SEQ ID NO: 7, and CDRH3 consisting of the amino acid sequence represented by SEQ ID NO: 3, and a light chain comprising CDRL1 consisting of the amino acid sequence represented by SEQ ID NO: 4, CDRL2 consisting of the amino acid sequence represented by SEQ ID NO: 5, and CDRL3 consisting of the amino acid sequence represented by SEQ ID NO: 6.
[31] The treatment method according to any one of [20] to [29], wherein the anti-MUC1 antibody is an antibody comprising a heavy chain comprising CDRH1 consisting of the amino acid sequence represented by SEQ ID NO: 1, CDRH2 consisting of the amino acid sequence represented by SEQ ID NO: 2, and CDRH3 consisting of the amino acid sequence represented by SEQ ID NO: 3, and a light chain comprising CDRL1 consisting of the amino acid sequence represented by SEQ ID NO: 4, CDRL2 consisting of the amino acid sequence represented by SEQ ID NO: 5, and CDRL3 consisting of the amino acid sequence represented by SEQ ID NO: 6.
[32] The treatment method according to any one of [20] to [29], wherein the anti-MUC1 antibody is an antibody comprising a heavy chain comprising a heavy chain variable region consisting of the amino acid sequence represented by SEQ ID NO: 8 or SEQ ID NO: 10, and a light chain comprising a light chain variable region consisting of the amino acid sequence represented by SEQ ID NO: 9.
[33] The treatment method according to any one of [20] to [29], wherein the anti-MUC1 antibody is an antibody comprising a heavy chain comprising a heavy chain variable region consisting of the amino acid sequence represented by SEQ ID NO: 8, and a light chain comprising a light chain variable region consisting of the amino acid sequence represented by SEQ ID NO: 9.
[34] The treatment method according to any one of [20] to [29], wherein the anti-MUC1 antibody is an antibody comprising a heavy chain consisting of the amino acid sequence represented by SEQ ID NO: 11 or SEQ ID NO: 13, and a light chain consisting of the amino acid sequence represented by SEQ ID NO: 12.
[35] The treatment method according to any one of [20] to [29], wherein the anti-MUC1 antibody is an antibody comprising a heavy chain consisting of the amino acid sequence represented by SEQ ID NO: 11, and a light chain consisting of the amino acid sequence represented by SEQ ID NO: 12.
[36] The treatment method according to [34] or [35], wherein a lysine residue at the carboxyl terminus of the heavy chain of the anti-MUC1 antibody is deleted.
[37] The treatment method according to any one of [20] to [36], wherein the average number of units of the drug-linker conjugated per antibody molecule in the anti-MUC1 antibody-drug conjugate is in the range of from 7 to 8.
[38] The treatment method according to any one of [20] to [36], wherein the average number of units of the drug-linker conjugated per antibody molecule in the anti-MUC1 antibody-drug conjugate is in the range of from 7.5 to 8.
[39] An anti-MUC1 antibody-drug conjugate for the treatment of a cancer having low sensitivity to an existing anti-cancer agent.
[40] The anti-MUC1 antibody-drug conjugate according to [39], wherein the anti-cancer agent is an alkylating agent, a platinum-containing drug, an antifolate, a pyridine metabolism inhibitor, a purine metabolism inhibitor, a ribonucleotide reductase inhibitor, a nucleotide analog, a topoisomerase inhibitor, a microtubule polymerization inhibitor, a microtubule depolymerization inhibitor, an antitumor antibiotic, an anti-hormonal agent, an EGFR inhibitor, a BTK inhibitor, a BCR-ABL inhibitor, an ALK inhibitor, a HER2 inhibitor, an angiogenesis inhibitor, a PARP inhibitor, an mTOR inhibitor, a membrane differentiation antigen targeting drug, or an antibody-drug conjugate.

[41] The anti-MUC1 antibody-drug conjugate according to [39], wherein the anti-cancer agent is an antibody-drug conjugate.

[42] The anti-MUC1 antibody-drug conjugate according to [41], wherein the antibody-drug conjugate is an anti-TROP2 antibody-drug conjugate, an anti-Nectin-4 antibody-drug conjugate, or an anti-HER2 antibody-drug conjugate.

[43] The anti-MUC1 antibody-drug conjugate according to [41], wherein the antibody-drug conjugate is enfortumab vedotin, sacituzumab govitecan, datopotamab deruxtecan, trastuzumab emtansine, or trastuzumab deruxtecan.

[44] The anti-MUC1 antibody-drug conjugate according to any one of [39] to [43], wherein the cancer expresses TA-MUC1.

[45] The anti-MUC1 antibody-drug conjugate according to [44], wherein the cancer is selected from the group consisting of ovarian cancer, breast cancer, pancreatic cancer, lung cancer, colon cancer, gastric cancer, liver cancer, kidney cancer, blood cancer, endometrial cancer, thyroid cancer, leukemia, seminoma, melanoma, carcinoma, teratoma, lymphoma, sarcoma, mesothelioma, neuroblastoma, glioma, rectal cancer, adrenal cancer, skin cancer, brain cancer, uterine cervix cancer, gut cancer, intestinal cancer, head-and-neck cancer, alimentary canal cancer, lymph node cancer, esophageal cancer, colorectal cancer, ear, nose and throat (ENT) cancer, prostate cancer, bladder cancer, uterine cancer, biliary tract cancer, and metastases thereof.

[46] The anti-MUC1 antibody-drug conjugate according to [44], wherein the cancer is breast cancer, lung cancer, or bladder cancer.

[47] The anti-MUC1 antibody-drug conjugate according to any one of [39] to [46], wherein the anti-MUC1 antibody is an anti-TA-MUC1 antibody.

[48] The anti-MUC1 antibody-drug conjugate according to any one of [39] to [47], wherein the anti-MUC1 antibody-drug conjugate is an anti-MUC1 antibody-drug conjugate, in which a drug-linker represented by the following formula:

[Chem. 3]

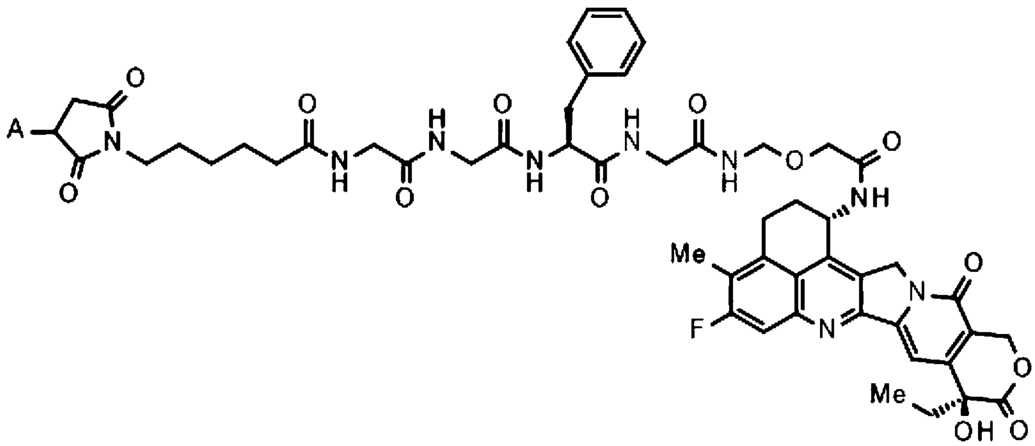

wherein A represents the connecting position to an anti-MUC1 antibody,
is conjugated to the anti-MUC1 antibody via a thioether bond.

[49] The anti-MUC1 antibody-drug conjugate according to any one of [39] to [48], wherein the anti-MUC1 antibody is an antibody comprising a heavy chain comprising CDRH1 consisting of the amino acid sequence represented by SEQ ID NO: 1, CDRH2 consisting of the amino acid sequence represented by SEQ ID NO: 2 or SEQ ID NO: 7, and CDRH3 consisting of the amino acid sequence represented by SEQ ID NO: 3, and a light chain comprising CDRL1 consisting of the amino acid sequence represented by SEQ ID NO: 4, CDRL2 consisting of the amino acid sequence represented by SEQ ID NO: 5, and CDRL3 consisting of the amino acid sequence represented by SEQ ID NO: 6.

[50] The anti-MUC1 antibody-drug conjugate according to any one of [39] to [48], wherein the anti-MUC1 antibody is an antibody comprising a heavy chain comprising CDRH1 consisting of the amino acid sequence represented by SEQ ID NO: 1, CDRH2 consisting of the amino acid sequence represented by SEQ ID NO: 2, and CDRH3 consisting of the amino acid sequence represented by SEQ ID NO: 3, and a light chain comprising CDRL1 consisting of the amino acid sequence represented by SEQ ID NO: 4, CDRL2 consisting of the amino acid sequence represented by SEQ ID NO: 5,

and CDRL3 consisting of the amino acid sequence represented by SEQ ID NO: 6.

[51] The anti-MUC1 antibody-drug conjugate according to any one of [39] to [48], wherein the anti-MUC1 antibody is an antibody comprising a heavy chain comprising a heavy chain variable region consisting of the amino acid sequence represented by SEQ ID NO: 8 or SEQ ID NO: 10, and a light chain comprising a light chain variable region consisting of the amino acid sequence represented by SEQ ID NO: 9.

[52] The anti-MUC1 antibody-drug conjugate according to any one of [39] to [48], wherein the anti-MUC1 antibody is an antibody comprising a heavy chain comprising a heavy chain variable region consisting of the amino acid sequence represented by SEQ ID NO: 8, and a light chain comprising a light chain variable region consisting of the amino acid sequence represented by SEQ ID NO: 9.

[53] The anti-MUC1 antibody-drug conjugate according to any one of [39] to [48], wherein the anti-MUC1 antibody is an antibody comprising a heavy chain consisting of the amino acid sequence represented by SEQ ID NO: 11 or SEQ ID NO: 13, and a light chain consisting of the amino acid sequence represented by SEQ ID NO: 12.

[54] The anti-MUC1 antibody-drug conjugate according to any one of [39] to [48], wherein the anti-MUC1 antibody is an antibody comprising a heavy chain consisting of the amino acid sequence represented by SEQ ID NO: 11, and a light chain consisting of the amino acid sequence represented by SEQ ID NO: 12.

[55] The anti-MUC1 antibody-drug conjugate according to [53] or [54], wherein a lysine residue at the carboxyl terminus of the heavy chain of the anti-MUC1 antibody is deleted.

[56] The anti-MUC1 antibody-drug conjugate according to any one of [39] to [55], wherein the average number of units of the drug-linker conjugated per antibody molecule in the anti-MUC1 antibody-drug conjugate is in the range of from 7 to 8.

[57] The anti-MUC1 antibody-drug conjugate according to any one of [39] to [55], wherein the average number of units of the drug-linker conjugated per antibody molecule in the anti-MUC1 antibody-drug conjugate is in the range of from 7.5 to 8.

[58] Use of an anti-MUC1 antibody-drug conjugate for the manufacture of a medicament for the treatment of a cancer having low sensitivity to an existing anti-cancer agent.

[59] The use according to [58], wherein the anti-cancer agent is an alkylating agent, a platinum-containing drug, an antifolate, a pyridine metabolism inhibitor, a purine metabolism inhibitor, a ribonucleotide reductase inhibitor, a nucleotide analog, a topoisomerase inhibitor, a microtubule polymerization inhibitor, a microtubule depolymerization inhibitor, an antitumor antibiotic, an anti-hormonal agent, an EGFR inhibitor, a BTK inhibitor, a BCR-ABL inhibitor, an ALK inhibitor, a HER2 inhibitor, an angiogenesis inhibitor, a PARP inhibitor, an mTOR inhibitor, a membrane differentiation antigen targeting drug, or an antibody-drug conjugate.

[60] The use according to [58], wherein the anti-cancer agent is an antibody-drug conjugate.

[61] The use according to [60], wherein the antibody-drug conjugate is an anti-TROP2 antibody-drug conjugate, an anti-Nectin-4 antibody-drug conjugate, or an anti-HER2 antibody-drug conjugate.

[62] The use according to [60], wherein the antibody-drug conjugate is enfortumab vedotin, sacituzumab govitecan, datopotamab deruxtecan, trastuzumab emtansine, or trastuzumab deruxtecan.

[63] The use according to any one of [58] to [62], wherein the cancer expresses TA-MUC1.

[64] The use according to [63], wherein the cancer is selected from the group consisting of ovarian cancer, breast cancer, pancreatic cancer, lung cancer, colon cancer, gastric cancer, liver cancer, kidney cancer, blood cancer, endometrial cancer, thyroid cancer, leukemia, seminoma, melanoma, carcinoma, teratoma, lymphoma, sarcoma, mesothelioma, neuroblastoma, glioma, rectal cancer, adrenal cancer, skin cancer, brain cancer, uterine cervix cancer, gut cancer, intestinal cancer, head-and-neck cancer, alimentary canal cancer, lymph node cancer, esophageal cancer, colorectal cancer, ear, nose and throat (ENT) cancer, prostate cancer, bladder cancer, uterine cancer, biliary tract cancer, and metastases thereof.

[65] The use according to [63], wherein the cancer is breast cancer, lung cancer, or bladder cancer.

[66] The use according to any one of [58] to [65], wherein the anti-MUC1 antibody is an anti-TA-MUC1 antibody.

[67] The use according to any one of [58] to [66], wherein the anti-MUC1 antibody-drug conjugate is an anti-MUC1 antibody-drug conjugate, in which a drug-linker represented by the following formula:

[Chem. 4]

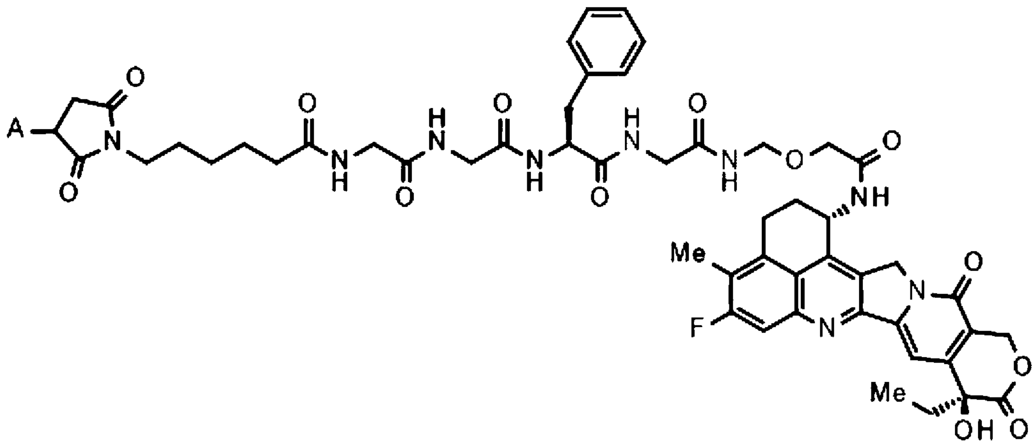

wherein A represents the connecting position to an anti-MUC1 antibody,
is conjugated to the anti-MUC1 antibody via a thioether bond.

[68] The use according to any one of [58] to [67], wherein the anti-MUC1 antibody is an antibody comprising a heavy chain comprising CDRH1 consisting of the amino acid sequence represented by SEQ ID NO: 1, CDRH2 consisting of the amino acid sequence represented by SEQ ID NO: 2 or SEQ ID NO: 7, and CDRH3 consisting of the amino acid sequence represented by SEQ ID NO: 3, and a light chain comprising CDRL1 consisting of the amino acid sequence represented by SEQ ID NO: 4, CDRL2 consisting of the amino acid sequence represented by SEQ ID NO: 5, and CDRL3 consisting of the amino acid sequence represented by SEQ ID NO: 6.
[69] The use according to any one of [58] to [67], wherein the anti-MUC1 antibody is an antibody comprising a heavy chain comprising CDRH1 consisting of the amino acid sequence represented by SEQ ID NO: 1, CDRH2 consisting of the amino acid sequence represented by SEQ ID NO: 2, and CDRH3 consisting of the amino acid sequence represented by SEQ ID NO: 3, and a light chain comprising CDRL1 consisting of the amino acid sequence represented by SEQ ID NO: 4, CDRL2 consisting of the amino acid sequence represented by SEQ ID NO: 5, and CDRL3 consisting of the amino acid sequence represented by SEQ ID NO: 6.
[70] The use according to any one of [58] to [67], wherein the anti-MUC1 antibody is an antibody comprising a heavy chain comprising a heavy chain variable region consisting of the amino acid sequence represented by SEQ ID NO: 8 or SEQ ID NO: 10, and a light chain comprising a light chain variable region consisting of the amino acid sequence represented by SEQ ID NO: 9.
[71] The use according to any one of [58] to [67], wherein the anti-MUC1 antibody is an antibody comprising a heavy chain comprising a heavy chain variable region consisting of the amino acid sequence represented by SEQ ID NO: 8, and a light chain comprising a light chain variable region consisting of the amino acid sequence represented by SEQ ID NO: 9.
[72] The use according to any one of [58] to [67], wherein the anti-MUC1 antibody is an antibody comprising a heavy chain consisting of the amino acid sequence represented by SEQ ID NO: 11 or SEQ ID NO: 13, and a light chain consisting of the amino acid sequence represented by SEQ ID NO: 12.
[73] The use according to any one of [58] to [67], wherein the anti-MUC1 antibody is an antibody comprising a heavy chain consisting of the amino acid sequence represented by SEQ ID NO: 11, and a light chain consisting of the amino acid sequence represented by SEQ ID NO: 12.
[74] The use according to [72] or [73], wherein a lysine residue at the carboxyl terminus of the heavy chain of the anti-MUC1 antibody is deleted.
[75] The use according to any one of [58] to [74], wherein the average number of units of the drug-linker conjugated per antibody molecule in the anti-MUC1 antibody-drug conjugate is in the range of from 7 to 8.
[76] The use according to any one of [58] to [74], wherein the average number of units of the drug-linker conjugated per antibody molecule in the anti-MUC1 antibody-drug conjugate is in the range of from 7.5 to 8.

## Advantageous Effects of Invention

The present invention can provide a treatment agent and a treatment method for a cancer having low sensitivity to an existing anti-cancer agent, comprising administering an anti-MUC1 antibody-drug conjugate.

Brief Description of Drawings

[Figure 1] Figure 1 is a diagram showing antitumor effects of TA-MUC1-DXd ADC, Control IgG-DXd ADC, Nectin-4-MMAE ADC, and TROP2-SN38 ADC on a bladder cancer PDX model (NIBIO-K071).

[Figure 2] Figure 2 is a diagram showing antitumor effects of TA-MUC1-DXd ADC, Control IgG-DXd ADC, and TROP2-SN38 ADC on a model with a transplanted breast cancer cell line HCC70.

[Figure 3] Figure 3 is a diagram showing an amino acid sequence of a heavy chain of PM-N54Q (SEQ ID NO: 11).

[Figure 4] Figure 4 is a diagram showing an amino acid sequence of a light chain of PM-N54Q and PankoMab-GEX(R) (SEQ ID NO: 12).

[Figure 5] Figure 5 is a diagram showing an amino acid sequence of a heavy chain of PankoMab-GEX(R) (SEQ ID NO: 13).

[Figure 6] Figure 6 is a diagram showing antitumor effects of TA-MUC1-DXd ADC and TROP2-DXd ADC on a lung cancer PDX model (NIBIO-NS1).

[Figure 7] Figure 7 is a diagram showing antitumor effects of TA-MUC1-DXd ADC, Control IgG-DXd ADC, and HER2-DXd ADC on a model with a transplanted breast cancer cell line HCC70.

Description of Embodiments

Hereinafter, preferred modes for carrying out the present invention are described. The embodiments described below are given merely for illustrating one example of a typical embodiment of the present invention and are not intended to limit the scope of the present invention.

As used herein, the term "low sensitivity" to an anti-cancer agent is not particularly limited, and means that cell growth is not eliminated by the anti-cancer agent, and also includes "no sensitivity". Cases where cancer cells, after being clinically treated with an anti-cancer agent, neither disappear nor shrink so that neither complete response (CR) nor partial response (PR) can be obtained, can be defined as having "low sensitivity". Cases where cancer cells, even though initially sensitive to an anti-cancer agent, acquire resistance by continuous administration of the anti-cancer agent, can also be defined as having "low sensitivity". Alternatively, cases where a cancer cell line causes no or only weak inhibition of cell growth under anti-cancer agent addition conditions may be defined as having "low sensitivity".

Besides, cases where cells exhibit a growth inhibition rate of less than 12.5%, 25%, or 50% at a drug concentration of 100 nM can be defined as having "low sensitivity". In the present invention, the "cancer" includes, particularly, ovarian cancer, breast cancer, pancreatic cancer, lung cancer, colon cancer, gastric cancer, liver cancer, kidney cancer, blood cancer, endometrial cancer, thyroid cancer, leukemia, seminoma, melanoma, carcinoma, teratoma, lymphoma, sarcoma, mesothelioma, neuroblastoma, glioma, rectal cancer, adrenal cancer, skin cancer, brain cancer, uterine cervix cancer, gut cancer, intestinal cancer, head-and-neck cancer, alimentary canal cancer, lymph node cancer, esophageal cancer, colorectal cancer, ear, nose and throat (ENT) cancer, prostate cancer, bladder cancer, uterine cancer, biliary tract cancer, and metastases thereof. The term cancer according to the present invention includes cancer metastasis.

As used herein, the term "tumor" means a group of cells or tissue that is formed by misregulated cell growth. The tumor may show partial or complete lack of structural organization and functional coordination with a normal tissue, and usually forms a distinct mass of tissue, which may be either benign or malignant. The terms "tumor" and "cancer" are interchangeably used.

As used herein, the term "metastasis" means the spread of cancer cells from the site of primary tumor to a distant site in the body. The metastasis is formed by a very complicated process and usually includes dissociation of cancer cells from the primary tumor, intravasation and circulation, and colonization in distant, normal tissues of the body and proliferation. When tumor cells metastasize, a new tumor is called secondary tumor or metastatic tumor, and the cells are usually similar to those in the primary tumor. This means that, for example, when breast cancer metastasizes to the lung, a secondary tumor is composed of aberrant breast cells, not aberrant lung cells. In this case, the tumor in the lung is called metastatic breast cancer, not lung cancer.

As used herein, the term "existing anti-cancer agent" refers to an anti-cancer agent that is clinically used except for the anti-MUC1 antibody-drug conjugate used in the present invention, and preferably refers to an anti-cancer agent that is used in standard treatment. The "existing anti-cancer agent" is not particularly limited as long as the requirements described above are satisfied. Examples thereof include alkylating agents, platinum-containing drugs, antimetabolic agents (e.g., antifolates, pyridine metabolism inhibitors, purine metabolism inhibitors, ribonucleotide reductase inhibitors, and nucleotide analogs), topoisomerase inhibitors, microtubule polymerization inhibitors, microtubule depolymerization inhibitors, antitumor antibiotics, anti-hormonal agents, and molecular targeting drugs (e.g., EGFR inhibitors, BTK inhibitors, BCR-ABL inhibitors, ALK inhibitors, HER2 inhibitors, angiogenesis inhibitors, PARP inhibitors, mTOR inhibitors, membrane differentiation antigen targeting drugs, and antibody-drug conjugates).

Examples of the alkylating agent include dacarbazine, nimustine, temozolomide, fotemustine, cyclophosphamide, and ifosfamide.

Examples of the platinum-containing drug include cisplatin, carboplatin, and oxaliplatin.

Examples of the antifolate include pemetrexed, leucovorin, and methotrexate.

Examples of the pyridine metabolism inhibitor include TS-1(R), 5-fluorouracil, UFT, carmofur, doxifluridine, and capecitabine.

Examples of the purine metabolism inhibitor include 6-mercaptopurine and azathioprine.

Examples of the ribonucleotide reductase inhibitor include hydroxycarbamide.

Examples of the nucleotide analog include gemcitabine.

Examples of the topoisomerase inhibitor include irinotecan and etoposide.

Examples of the microtubule polymerization inhibitor include vincristine, vinblastine, vinorelbine, and eribulin.

Examples of the microtubule depolymerization inhibitor include docetaxel and paclitaxel.

Examples of the antitumor antibiotic include doxorubicin, bleomycin, mitomycin C, and epirubicin.

Examples of the anti-hormonal agent include tamoxifen, fulvestrant, goserelin, leuprorelin, anastrozole, letrozole, and exemestane.

Examples of the EGFR inhibitor include osimertinib, afatinib, gefitinib, erlotinib, cetuximab, and panitumumab.

Examples of the BTK inhibitor include ibrutinib, acalabrutinib, and tirabrutinib.

Examples of the BCR-ABL inhibitor include imatinib, dasatinib, and nilotinib.

Examples of the ALK inhibitor include crizotinib.

Examples of the HER2 inhibitor include trastuzumab, pertuzumab, and lapatinib.

Examples of the angiogenesis inhibitor include regorafenib, axitinib, sorafenib, sunitinib, pazopanib, ramucirumab, and bevacizumab.

Examples of the PARP inhibitor include olaparib, rucaparib, niraparib, talazoparib, and veliparib.

Examples of the mTOR inhibitor include temsirolimus and everolimus.

Examples of the membrane differentiation antigen targeting drug include ibritumomab tiuxetan, ofatumumab, rituximab, brentuximab vedotin, gemtuzumab ozogamicin, and mogamulizumab.

Examples of the antibody-drug conjugate include anti-HER2 antibody-drug conjugates such as trastuzumab emtansine and trastuzumab deruxtecan, anti-Nectin-4 antibody-drug conjugates such as enfortumab vedotin, and anti-TROP2 antibody-drug conjugates such as sacituzumab govitecan and datopotamab deruxtecan.

Examples of the cell line corresponding to the cancer having low sensitivity to the existing anti-cancer agent can include NIBIO-K071, HCC70, and NIBIO-NS1. The cell line corresponding to the cancer having low sensitivity to the existing anti-cancer agent can be selected, for example, by confirming the presence or absence of positive findings by a method described in Example 2 and Example 3 using an arbitrary cancer cell line.

The antitumor effect of a drug for the cancer having low sensitivity to the existing anti-cancer agent can be confirmed by, for example, *in vitro* cell growth inhibitory activity against the cell line, or the *in vivo* tumor growth inhibition rate in a model in which the cell line has been transplanted in a nude mouse.

As used herein, the term "patient" means a human, a non-human primate, or other animals, particularly, mammals such as a bovine, a horse, a pig, a sheep, a goat, a dog, a cat, or rodents such as a mouse and a rat. In a particularly preferable embodiment, the patient is a human.

As used herein, the term "MUC1" refers to a protein MUC1 also known as mucin-1, polymorphic epithelial mucin (PEM), or cancer antigen 15-3, particularly, human MUC1 (accession No. P15941). MUC1, a member of the mucin family, encodes membrane-bound glycosylated phosphoprotein. MUC1 possesses a core protein mass of 120 to 225 kDa, and this mass increases to 250 to 500 kDa with glycosylation. MUC1 protrudes 200 to 500 nm above the cell surface. This protein is anchored by the transmembrane domain to the apical surface of many epithelial cells. The extracellular domain contains 20 amino acid variable number tandem repeat (VNTR) domains, and the number of repeats ranges from 25 to 120 repeats in different individuals. Such repeats are rich in serine, threonine, and proline residues, which permit a high level of O-glycosylation. In certain embodiments, the term "MUC1" refers to tumor-associated MUC1 ("TA-MUC1"). TA-MUC1 is MUC1 present on cancer cells. This MUC1 differs from MUC1 present on non-cancer cells in that TA-MUC1 has a much higher expression level, is localized, and has a distinct glycosylation pattern. Particularly, TA-MUC1 is present over the entire surface of the cancer cell without polarity while MUC1 on a non-cancer cell strictly exhibits apical expression and therefore does not permit access of a systemically administered antibody. Furthermore, TA-MUC1 has aberrant O-glycosylation that exposes a new peptide epitope in the MUC1 protein backbone, and new hydrocarbon tumor antigens such as N-acetylgalactosamine (Tn), sialyl $\alpha$2-6N-acetylgalactosamine (sTn), galactose $\beta$1-3N-acetylgalactosamine (TF), or galactose $\beta$1-3 (sialyl $\alpha$2-6)N-acetylgalactosamine (sTF).

As used herein, the term "antibody" refers to a protein containing at least two heavy chains and two light chains connected through disulfide bonds. The antibody may be, for example, a humanized antibody, a human antibody, or a chimeric antibody. The antibody includes multivalent and multispecific antibodies, that is, an antibody construct having

more than two binding sites, each of which binds to the same epitope, and an antibody construct having one or more binding sites that bind to a first epitope, one or more binding sites that bind to a second epitope, and even an optionally selected further binding site that binds to a further epitope.

As used herein, the term "anti-MUC1 antibody" refers to an antibody that specifically binds to MUC1, and has an activity of internalizing in MUC1-expressing cells by binding to MUC1, in other words, an activity of migrating into MUC1-expressing cells after binding to MUC1.

As used herein, the term "specific binding" preferably means that an antibody binds more strongly to a specific target such as an epitope than it binds to other targets. Examples of criteria for determining whether or not binding is specific can include dissociation constants (in the present specification, referred to as "$K_d$"). An antibody, when binding to a first target with a lower dissociation constant ($K_d$) than that for a second target, binds more strongly to the first target than it binds to the second target. Preferably, the dissociation constant for a target to which an antibody specifically binds is 100 times, 200 times, 500 times, or 1000 times lower than that of a target to which the antibody does not specifically bind. The term "specific binding" further refers to, particularly, binding affinity between binding partners, and its affinity constant $K_a$ is at least $10^6$ $M^{-1}$, preferably at least $10^7$ $M^{-1}$, and more preferably at least $10^8$ $M^{-1}$. An antibody specific for a certain antigen refers to an antibody capable of binding to the antigen, particularly, with affinity having $K_a$ of at least $10^6$ $M^{-1}$, preferably at least $10^7$ $M^{-1}$, and more preferably at least $10^8$ $M^{-1}$. The term "anti-MUC1 antibody" as used herein refers to an antibody capable of specifically binding to MUC1 and preferably binding to MUC1 with an affinity having $K_a$ of at least $10^6$ $M^{-1}$, preferably at least $10^7$ $M^{-1}$, and more preferably at least $10^8$ $M^{-1}$.

The anti-MUC1 antibody used in the present invention specifically binds to an epitope on MUC1. The epitope resides in an extracellular tandem repeat of MUC1. The anti-MUC1 antibody used in the present invention binds to MUC1 in a glycosylation-dependent manner. Particularly, when a threonine residue of the tandem repeat is glycosylated with N-acetylgalactosamine (Tn), sialyl $\alpha$2-6N-acetylgalactosamine (sTn), galactose $\beta$1-3N-acetylgalactosamine (TF), or galactose $\beta$1-3 (sialyl $\alpha$2-6)N-acetylgalactosamine (sTF), preferably with Tn or TF, the anti-MUC1 antibody binds more strongly to MUC1. Preferably, the hydrocarbon moiety is bound to the threonine residue through an $\alpha$-O-glycoside bond. The epitope in the tandem repeat domain of MUC1 particularly comprises an amino acid sequence PDTR (SEQ ID NO: 16) or PESR (SEQ ID NO: 17). Binding to this epitope preferably depends on glycosylation as described above, and the binding increases, particularly, when the hydrocarbon moiety described above is attached to the threonine residue of the sequence PDTR or PESR.

The epitope for the anti-MUC1 antibody used in the present invention is preferably a tumor-associated MUC1 epitope (TA-MUC1). The TA-MUC1 epitope refers to an MUC1 epitope that is present on tumor cells but is, however, not present on normal cells, and/or permits access of an antibody circulating in a host only when present on tumor cells and permits no access thereof when present on normal cells. The epitope for the anti-MUC1 antibody used in the present invention is more preferably an epitope that comprises at least one PDTR sequence of the MUC1 tandem repeat and is glycosylated at threonine of the PDTR sequence with N-acetylgalactosamine (Tn) or galactose $\beta$1-3N-acetylgalactosamine (TF), preferably through an $\alpha$-O-glycoside bond.

The anti-MUC1 antibody used in the present invention can be obtained by procedures known in the art. For example, the antibody of the present invention can be obtained using a method usually carried out in the art, which involves immunizing animals with antigenic MUC1 or an arbitrary polypeptide selected from the amino acid sequence of MUC1, or a human-derived cancer cell line and collecting and purifying antibodies produced *in vivo.* The origin of the antigen is not limited to humans, and the animals may be immunized with an antigen derived from a non-human animal such as a mouse, a rat or the like.

Alternatively, antibody-producing cells which produce antibodies against the antigen are fused with myeloma cells according to methods known in the art (e.g., Kohler and Milstein, Nature (1975) 256, p. 495-497; and Kennet, R. ed., Monoclonal Antibodies, p. 365-367, Plenum Press, N.Y. (1980)) to establish hybridomas, from which monoclonal antibodies can in turn be obtained.

The antigen can be obtained by genetically engineering host cells to produce a gene encoding the antigenic protein. Specifically, vectors that permit expression of the antigen gene are prepared and transferred to host cells so that the gene is expressed. The antigen thus expressed can be purified. The antibody can also be obtained by a method of immunizing animals with the above-described genetically engineered antigen-expressing cells or a cell line expressing the antigen.

The anti-MUC1 antibody used in the present invention is preferably a recombinant antibody obtained by artificial modification for the purpose of decreasing heterologous antigenicity to humans such as a chimeric antibody or a humanized antibody, or is preferably an antibody having only the gene sequence of an antibody derived from a human, that is, a human antibody. These antibodies can be produced using known methods.

Examples of the chimeric antibody can include an antibody in which antibody variable and constant regions are derived from different species, for example, a chimeric antibody in which a mouse- or rat-derived antibody variable region is connected to a human-derived antibody constant region (Proc. Natl. Acad. Sci. USA, 81, 6851-6855, (1984)).

Examples of the humanized antibody can include an antibody obtained by integrating only the complementarity

determining region (CDR) of a heterologous antibody into a human-derived antibody (Nature (1986) 321, pp. 522-525), and an antibody obtained by grafting some of the amino acid residues of the framework of a heterologous antibody as well as the CDR sequence of the heterologous antibody to a human antibody by a CDR-grafting method (WO 90/07861), and an antibody humanized using a gene conversion mutagenesis strategy (U.S. Patent No. 5821337).

Examples of the human antibody can include an antibody generated by using a human antibody-producing mouse having a human chromosome fragment including genes of a heavy chain and light chain of a human antibody (see Tomizuka, K. et al., Nature Genetics (1997) 16, p. 133-143; Kuroiwa, Y. et. al., Nucl. Acids Res. (1998) 26, p. 3447-3448; Yoshida, H. et. al., Animal Cell Technology: Basic and Applied Aspects vol. 10, p. 69-73 (Kitagawa, Y., Matsuda, T. and Iijima, S. eds.), Kluwer Academic Publishers, 1999; Tomizuka, K. et. al., Proc. Natl. Acad. Sci. USA (2000) 97, p. 722-727, etc.). Alternatively, examples of the human antibody can include an antibody obtained by phage display, the antibody being selected from a human antibody library (see Wormstone, I. M. et. al, Investigative Ophthalmology & Visual Science.

(2002)43 (7), p. 2301-2308; Carmen, S. et. al., Briefings in Functional Genomics and Proteomics (2002), 1 (2), p. 189-203; Siriwardena, D. et. al., Ophthalmology (2002) 109 (3), p. 427-431, etc.).

The anti-MUC1 antibody used in the present invention also includes an antibody derived from the chimeric antibody, the humanized antibody, or the human antibody by the substitution of 1 to 3 amino acid residues in the CDRs by other amino acid residues as long as the antibody has the ability to bind specifically to the epitope for anti-MUC1. These antibodies can be produced using known methods.

In the present invention, modified variants of the anti-MUC1 antibody used in the present invention are also included. The modified variant refers to a variant obtained by subjecting the antibody according to the present invention to chemical or biological modification. Examples of the chemically modified variant include variants including a linkage of a chemical moiety to an amino acid skeleton, variants including a linkage of a chemical moiety to an N-linked or O-linked carbohydrate chain, etc. Examples of the biologically modified variant include variants obtained by post-translational modification (such as N-linked or O-linked glycosylation, N- or C-terminal processing, deamidation, isomerization of aspartic acids, or oxidation of methionine), and variants in which a methionine residue has been added to the N terminus by being expressed in a prokaryotic host cell. Further, an antibody labeled so as to enable the detection or isolation of the anti-MUC1 antibody or antigen used in the present invention, for example, an enzyme-labeled antibody, a fluorescence-labeled antibody, and an affinity-labeled antibody are also included in the meaning of the modified variant. Such a modified variant of the anti-MUC1 antibody used in the present invention is useful for improving the stability and blood retention of the antibody, reducing the antigenicity thereof, detecting or isolating an antibody or an antigen, and so on.

It is known that a lysine residue at the carboxyl terminus of the heavy chain of an antibody produced in a cultured mammalian cell is deleted (Journal of Chromatography A, 705: 129-134 (1995)), and it is also known that two amino acid residues (glycine and lysine) at the carboxyl terminus of the heavy chain of an antibody produced in a cultured mammalian cell are deleted and a proline residue newly located at the carboxyl terminus is amidated (Analytical Biochemistry, 360: 75-83 (2007)). However, such deletion and modification of the heavy chain sequence do not affect the antigen-binding affinity and the effector function (the activation of complement, antibody-dependent cellular cytotoxicity, etc.) of the antibody. Therefore, in the anti-MUC1 antibody used in the present invention, antibodies subjected to such modification and functional fragments of the antibody are also included, and deletion variants in which one or two amino acids have been deleted at the carboxyl terminus of the heavy chain, variants obtained by amidation of deletion variants (for example, a heavy chain in which the carboxyl terminal proline residue has been amidated), and the like are also included. The two heavy chains constituting the anti-MUC1 antibody used in the present invention may be of one type selected from the group consisting of a full-length heavy chain and the above-described deletion variant, or may be of two types in combination selected therefrom. The ratio of the amount of each deletion variant can be affected by the type of cultured mammalian cells which produce the anti-MUC1 antibody used in the present invention and the culture conditions; however, examples of the anti-MUC1 antibody used in the present invention can preferably include an antibody in which one amino acid residue at the carboxyl terminus has been deleted in both of the two heavy chains.

Examples of isotypes of the anti-MUC1 antibody used in the present invention can include, for example, humanized or human IgG (IgG1, IgG2, IgG3, IgG4), and preferably IgG1 or IgG4, and more preferably, the isotype is IgG1. Alternatively, their variants may be used as the anti-MUC1 antibody according to the present invention.

Examples of the anti-MUC1 antibody used in the present invention can include PankoMab-GEX(R) (International Publication No. WO 2011/012309), PM-N54Q (International Publication No. WO 2019/219889), and its variants, active fragments, and modified variants and can preferably include PM-N54Q. These anti-MUC1 antibodies can be produced by methods described in the literature. The amino acid sequences of a heavy chain and a light chain of PankoMab-GEX(R) are shown in SEQ ID NO: 13 (Figure 5) and SEQ ID NO: 12 (Figure 4), respectively. The amino acid sequences of a heavy chain and a light chain of PM-N54Q are shown in SEQ ID NO: 11 (Figure 3) and SEQ ID NO: 12 (Figure 4), respectively.

As used herein, the term "antibody-drug conjugate" refers to a conjugate of a drug with cytotoxicity conjugated to an antibody via a linker. Examples of the antibody-drug conjugate can include those described in U.S. Patent No. 7097840, U.S. Patent No. 7999083, International Publication No. WO 2010/093395, International Publication No. WO 2014/057687, International Publication No. WO 2015/115091, International Publication No. WO 2017/083582, and International

Publication No. WO 2019/219891, preferably those described in International Publication No. WO 2019/219891. These antibody-drug conjugates can be produced by methods described in the literature.

The drug with cytotoxicity is not particularly limited as long as the drug has an antitumor effect and has a substituent or a partial structure that can be connected to a linker. Examples thereof can include camptothecin, calicheamicin, doxorubicin, daunorubicin, mitomycin C, bleomycin, cyclocytidine, vincristine, vinblastine, methotrexate, cisplatin, auristatin E, maytansine, paclitaxel, pyrrolobenzodiazepine, and their derivatives, and can preferably include camptothecin derivatives, more preferably exatecan derivatives.

As used herein, the term "drug-linker" refers to a drug and a linker moiety in the antibody-drug conjugate, in other words, a partial structure of the antibody-drug conjugate excluding the antibody.

As used herein, the term "anti-MUC1 antibody-drug conjugate" refers to an antibody-drug conjugate having an anti-MUC1 antibody as the antibody for the antibody-drug conjugate. Examples of the anti-MUC1 antibody-drug conjugate can include those described in International Publication No. WO 2019/219891, International Publication No. WO 2017/083582, and International Publication No. WO 2021/247798. These anti-MUC1 antibody-drug conjugates can be produced by methods described in the literature.

The anti-MUC1 antibody-drug conjugate more preferably used in the present invention is an anti-MUC1 antibody-drug conjugate, in which a drug-linker represented by the following formula:

[Chem. 5]

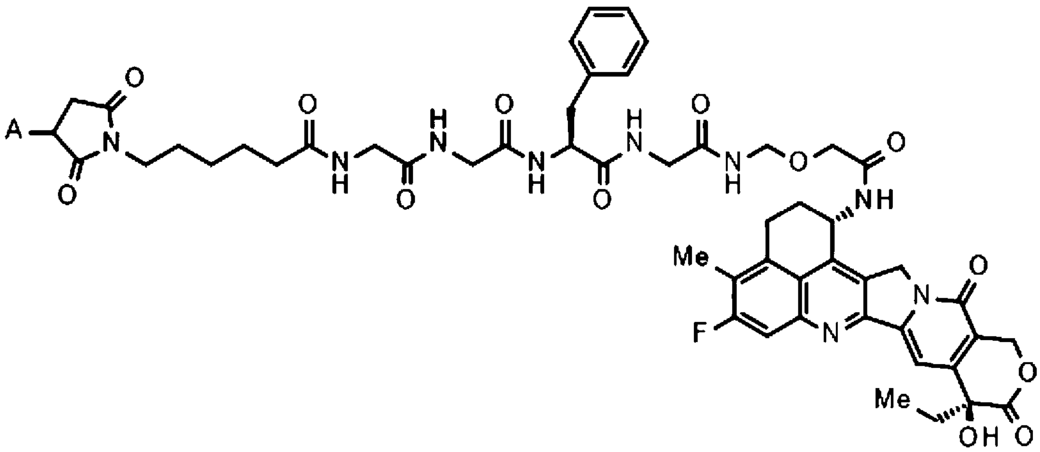

wherein A represents the connecting position to an anti-MUC1 antibody,
is conjugated to the anti-MUC1 antibody via a thioether bond. The drug-linker is connected to a thiol group (in other words, the sulfur atom of a cysteine residue) formed at an interchain disulfide bond site (two sites between heavy chains, and two sites between a heavy chain and a light chain) in the antibody.

The anti-MUC1 antibody-drug conjugate more preferably used in the present invention may be represented by the following formula:

[Chem. 6]

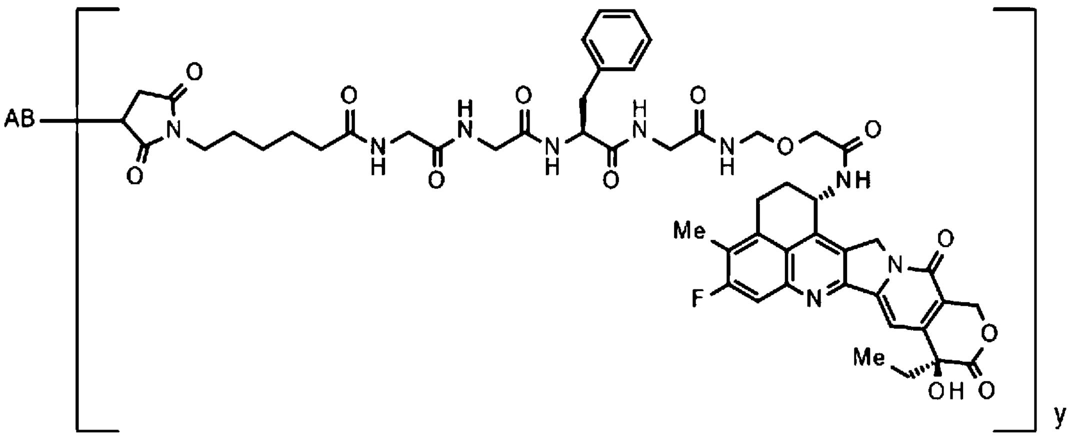

In this formula, AB represents an anti-MUC1 antibody, and the drug-linker is conjugated to the antibody via a thioether bond. The meaning of y is the same as that of what is called the average number of conjugated drug molecules (DAR; Drug-to-Antibody Ratio), and indicates the average number of units of the drug-linker conjugated per antibody molecule.

The anti-MUC1 antibody moiety of the anti-MUC1 antibody-drug conjugate used in the present invention is an antibody comprising a heavy chain comprising a complementarity determining region (CDR) CDRH1 having the amino acid sequence of SEQ ID NO: 1, CDRH2 having the amino acid sequence of SEQ ID NO: 2 or SEQ ID NO: 7, and CDRH3 having the amino acid sequence of SEQ ID NO: 3, and a light chain comprising a complementarity determining region (CDR) CDRL1 having the amino acid sequence of SEQ ID NO: 4, CDRL2 having the amino acid sequence 5, and CDRL3 having the amino acid sequence of SEQ ID NO: 6,

preferably an antibody comprising a heavy chain comprising CDRH1 consisting of the amino acid sequence represented by SEQ ID NO: 1, CDRH2 consisting of the amino acid sequence represented by SEQ ID NO: 2, and CDRH3 consisting of the amino acid sequence represented by SEQ ID NO: 3, and a light chain comprising CDRL1 consisting of the amino acid sequence represented by SEQ ID NO: 4, CDRL2 consisting of the amino acid sequence represented by SEQ ID NO: 5, and CDRL3 consisting of the amino acid sequence represented by SEQ ID NO: 6,
more preferably an antibody comprising a heavy chain comprising a heavy chain variable region consisting of the amino acid sequence represented by SEQ ID NO: 8 or SEQ ID NO: 10, and a light chain comprising a light chain variable region consisting of the amino acid sequence represented by SEQ ID NO: 9,
more preferably an antibody comprising a heavy chain comprising a heavy chain variable region consisting of the amino acid sequence represented by SEQ ID NO: 8, and a light chain comprising a light chain variable region consisting of the amino acid sequence represented by SEQ ID NO: 9,
more preferably an antibody comprising a heavy chain consisting of the amino acid sequence represented by SEQ ID NO: 11 or SEQ ID NO: 13, and a light chain consisting of the amino acid sequence represented by SEQ ID NO: 12, or an antibody in which a lysine residue at the carboxyl terminus of the heavy chain of the antibody is deleted, and
more preferably an antibody comprising a heavy chain consisting of the amino acid sequence represented by SEQ ID NO: 11, and a light chain consisting of the amino acid sequence represented by SEQ ID NO: 12, or an antibody in which a lysine residue at the carboxyl terminus of the heavy chain of the antibody is deleted.

A drug-linker intermediate for use in the production of the anti-MUC1 antibody-drug conjugate described above is represented by the following formula:

[Chem. 7]

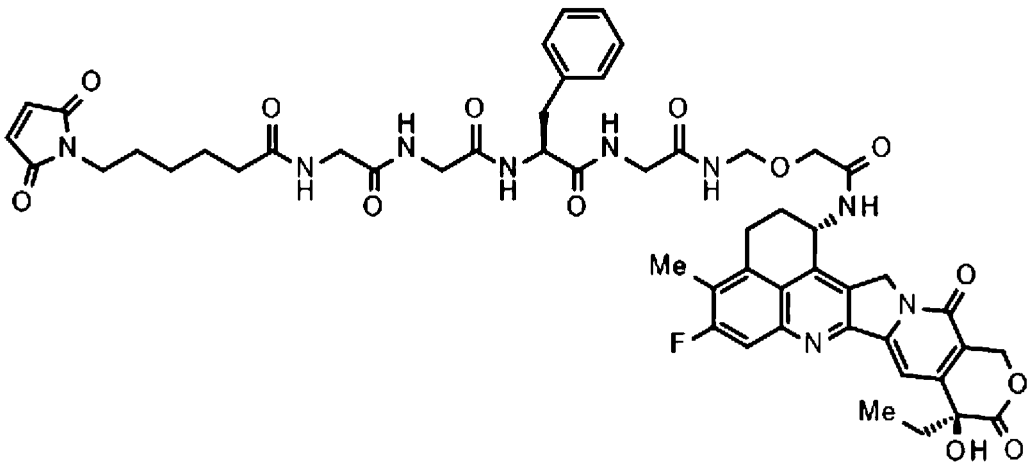

The drug-linker intermediate described above can be represented by the chemical name N-[6-(2,5-dioxo-2,5-dihydro-1H-pyrrol-1-yl)hexanoyl]glycylglycyl-L-phenylalanyl-N-[(2-{[(1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl]amino}-2-oxoethoxy)methyl]glycinamide, and can be produced with reference to descriptions in International Publication No. WO 2014/057687 and International Publication No. WO 2015/155998, and so on.

The anti-MUC1 antibody-drug conjugate preferably used in the present invention can be produced by reacting the drug-linker intermediate mentioned above and an anti-MUC1 antibody having a thiol group (alternatively referred to as a sulfhydryl group).

The anti-MUC1 antibody having a sulfhydryl group can be obtained by a method well known in the art (Hermanson, G. T, Bioconjugate Techniques, pp. 56-136, pp. 456-493, Academic Press (1996)). For example, by using 0.3 to 3 molar equivalents of a reducing agent such as tris(2-carboxyethyl)phosphine hydrochloride (TCEP) per interchain disulfide within the antibody and reacting with the anti-MUC1 antibody in a buffer solution containing a chelating agent such as ethylenediamine tetraacetic acid (EDTA), an anti-MUC1 antibody having a sulfhydryl group with partially or completely reduced interchain disulfides within the antibody can be obtained.

Further, by using 2 to 20 molar equivalents of the drug-linker intermediate per anti-MUC1 antibody having a sulfhydryl group, an anti-MUC1 antibody-drug conjugate in which 2 to 8 drug molecules are conjugated per antibody molecule can be produced.

The average number of conjugated drug molecules per antibody molecule of the anti-MUC1 antibody-drug conjugate produced can be determined, for example, by a method of calculation based on measurement of UV absorbance by the anti-MUC1 antibody-drug conjugate and the conjugation precursor thereof at two wavelengths of 280 nm and 370 nm (UV method), or a method of calculation based on quantification through HPLC measurement of fragments obtained by treating the antibody-drug conjugate with a reducing agent (HPLC method).

Conjugation between the anti-MUC1 antibody and the drug-linker intermediate and calculation of the average number of conjugated drug molecules per antibody molecule of the anti-MUC1 antibody-drug conjugate can be performed with reference to the description in International Publication No. WO 2015/155998, and so on.

The average number of units of the drug-linker conjugated per antibody molecule in the anti-MUC1 antibody-drug conjugate used in the present invention is preferably 2 to 8, more preferably 3 to 8, even more preferably 7 to 8, even more preferably 7.5 to 8, and even more preferably about 8. In other embodiments, the average number of units of the drug or the drug-linker conjugated per antibody molecule in the anti-MUC1 antibody-drug conjugate used in the present invention is an integer in the range of from preferably 2 to 8, more preferably 2, 4, 6, or 8, and even more preferably 8.

The treatment agent and the treatment method of the present invention comprises administering an anti-MUC1 antibody-drug conjugate and can be used for the treatment of a cancer having low sensitivity to an existing anti-cancer agent.

The "existing anti-cancer agent" in the "cancer having low sensitivity to an existing anti-cancer agent" is preferably an anti-TROP2 antibody-drug conjugate, an anti-Nectin-4 antibody-drug conjugate, or an anti-HER2 antibody-drug conjugate, more preferably enfortumab vedotin, sacituzumab govitecan, datopotamab deruxtecan, trastuzumab emtansine, or trastuzumab deruxtecan, and even more preferably datopotamab deruxtecan or sacituzumab govitecan.

The "cancer having low sensitivity to an existing anti-cancer agent" preferably expresses MUC1, more preferably

expresses TA-MUC1, and even more preferably highly expresses TA-MUC1. The expression of MUC1 can be confirmed by, for example, detection at the gene product (protein) level of MUC1 using an immunohistochemical method (IHC), a flow cytometer, Western blot, or the like, or detection at the transcription level of the gene by an *in situ* hybridization method (ISH) or quantitative PCR (q-PCR). Whether or not MUC1 is highly expressed can be determined using methods well known to those skilled in the art.

Examples of the cancer expressing MUC1 can include ovarian cancer, breast cancer, pancreatic cancer, lung cancer, colon cancer, gastric cancer, liver cancer, kidney cancer, blood cancer, endometrial cancer, thyroid cancer, leukemia, seminoma, melanoma, carcinoma, teratoma, lymphoma, sarcoma, mesothelioma, neuroblastoma, glioma, rectal cancer, adrenal cancer, skin cancer, brain cancer, uterine cervix cancer, gut cancer, intestinal cancer, head-and-neck cancer, alimentary canal cancer, lymph node cancer, esophageal cancer, colorectal cancer, ear, nose and throat (ENT) cancer, prostate cancer, bladder cancer, uterine cancer, biliary tract cancer, and metastases thereof. The cancer is preferably breast cancer, lung cancer, or bladder cancer.

The treatment agent and the treatment method of the present invention can preferably be used for administration to a patient having a history of treatment with an existing anti-cancer agent. The treatment agent and the treatment method of the present invention exhibit excellent antitumor activity against cancers having low sensitivity to an existing anti-cancer agent. Thus, the treatment agent and the treatment method of the present invention exert a marked antitumor effect when applied to a patient group having low sensitivity to an existing anti-cancer agent (patients having a history of treatment with an existing anti-cancer agent) among cancer patients. The "existing anti-cancer agent" is defined as mentioned above and is preferably an anti-TROP2 antibody-drug conjugate, an anti-Nectin-4 antibody-drug conjugate, or an anti-HER2 antibody-drug conjugate, more preferably enfortumab vedotin, sacituzumab govitecan, datopotamab deruxtecan, trastuzumab emtansine, or trastuzumab deruxtecan, even more preferably datopotamab deruxtecan or sacituzumab govitecan.

The treatment agent and the treatment method of the present invention may involve one or more additional drugs (e.g., a second drug) other than the anti-MUC1 antibody-drug conjugate used in the present invention. Specifically, the anti-MUC1 antibody-drug conjugate for use in the treatment agent and the treatment method of the present invention can be administered in combination with other drugs. The anti-cancer effect may be enhanced accordingly. Other drugs used for such a purpose may be administered concurrently with, separately from, or continuously with the anti-MUC1 antibody-drug conjugate used in the present invention, or these agents may be administered in a staggered manner. Other drugs are, or the second drug is, preferably an anti-cancer agent. Such an anti-cancer agent is not limited as long as the drug has antitumor activity. The anti-cancer agent is, for example, paclitaxel, docetaxel, SBT-1214, cyclophosphamide, imatinib, pazopanib, capecitabine, cytarabine, vinorelbine, gemcitabine, daunorubicin, doxorubicin, epirubicin, idarubicin, valrubicin, mitoxantrone, aminoglutethimide, testolactone, anastrozole, letrozole, exemestane, vorozole, formestane, fadrozole, 4-hydroxyandrostendione, 1,4,6-androstatriene-3,17-dione (ATD), 4-androstene-3,6,17-trione (6-oxo), irinotecan, topotecan, camptothecin, lamellarin D, etoposide, teniposide, mitoxantrone, amsacrine, ellipticine, aurintricarboxylic Acid, HU-331, cisplatin, carboplatin, oxaliplatin, olaparib, rucaparib, niraparib, imiquimod, resiquimod, methotrexate, pemetrexed, raltitrexed, pralatrexate, fluorouracil, gemcitabine, floxuridine, 5-fluorouracil, tegafur uracil, cetuximab, tomuzotuximab, panitumumab, zalutumumab, nimotuzumab, matuzumab, necitumumab, trastuzumab, timigutuzumab, pertuzumab, bevacizumab, alemtuzumab, brentuximab, gemtuzumab, rituximab, tositumomab, or ibritumomab, and is preferably paclitaxel, docetaxel, cisplatin, carboplatin, gemcitabine, or cetuximab.

The treatment agent and the treatment method of the present invention can be selected and used as an agent for drug therapy, which is the main method for treating cancer, and as a result, can delay development of cancer cells, inhibit growth thereof, and further kill cancer cells. These effects can allow cancer patients to be free from symptoms caused by cancer or achieve improvement in QOL of cancer patients and attain a therapeutic effect by sustaining the lives of the cancer patients. Even if the treatment agent and the treatment method of the present invention do not accomplish killing cancer cells, they can achieve higher QOL of cancer patients while achieving longer-term survival, by inhibiting or controlling the growth of cancer cells.

In such drug therapy, the treatment agent and the treatment method of the present invention can be used as an agent alone and in addition, the treatment agent and the treatment method may be used as an agent to be combined with an additional therapy in adjuvant therapy and can be combined with surgery, radiotherapy, hormone therapy, or the like. Furthermore, the treatment agent and the treatment method of the present invention can also be used as an agent for drug therapy in neoadjuvant therapy.

In addition to the therapeutic use as described above, a prophylactic effect such as suppressing the growth of small metastatic cancer cells and further killing them can, for example, also be expected for the treatment agent and the treatment method of the present invention. For example, an effect that inhibits and kills cancer cells in a body fluid in the course of metastasis or an effect that, for example, inhibits and kills small cancer cells immediately after implantation in any tissue can be expected. Accordingly, inhibition of cancer metastasis or a prophylactic effect can be expected, particularly, after surgical removal of a cancer.

The treatment agent and the treatment method of the present invention can be applied as systemic therapy to

patients as well as locally applied to cancer tissues to exert a therapeutic effect.

The treatment agent and the treatment method of the present invention can preferably be used for a mammal and can more preferably be used for a human.

The treatment agent of the present invention can be administered as a pharmaceutical composition containing at least one pharmaceutically suitable ingredient. The substances that may be used in the pharmaceutical composition of the present invention can be suitably selected and applied from formulation additives or the like that are generally used in the art, according to the dosage, administration concentration, and so on. The pharmaceutical composition typically contains, for example, at least one pharmaceutical carrier (e.g., a sterilized liquid). In this context, the liquid includes, for example, water and oil (petroleum and oil of animal origin, plant origin, or synthetic origin). The oil can be, for example, peanut oil, soybean oil, mineral oil, or sesame oil. Water is a more typical carrier when the pharmaceutical composition is intravenously administered. An aqueous saline solution, an aqueous dextrose solution, and an aqueous glycerol solution can also be used as a liquid carrier, in particular, for an injection solution. A suitable pharmaceutical vehicle can be appropriately selected from those known in the art. If desired, the pharmaceutical composition may also contain a trace amount of a moisturizing agent, an emulsifying agent, or a pH buffering agent. Examples of suitable pharmaceutical carriers are disclosed in "Remington’s Pharmaceutical Sciences" by E. W. Martin. The prescription corresponds to an administration mode.

Various delivery systems are known and can be used for administering the pharmaceutical composition of the present invention. Examples of the administration route can include, but are not limited to, intradermal, intramuscular, intraperitoneal, intravenous, and subcutaneous routes. The administration can be made for example by injection or bolus injection. In a particularly preferable embodiment, the administration of the antibody-drug conjugate is performed by injection. Parenteral administration is a preferable administration route.

In a representative embodiment, the pharmaceutical composition is prescribed, as a composition suitable for intravenous administration to a human, in accordance with conventional procedures. The composition for intravenous administration is typically a solution in a sterile and isotonic aqueous buffer solution. If necessary, the pharmaceutical composition may also contain a solubilizing agent and a local anesthetic to alleviate pain at an injection area (e.g., lignocaine). In general, the components described above are provided, either separately or together in a mixture in unit dosage form, as a freeze-dried powder or an anhydrous concentrate contained in a container which is obtained by sealing in e.g., an ampoule or a sachet indicating the amount of the active agent. When the pharmaceutical composition is in a form to be administered by injection, it may be administered using, for example, an injection bottle containing water or saline of sterile pharmaceutical grade. When the medicament is to be administered by injection, an ampoule of sterile water or saline for injection may be provided such that the components described above are admixed with each other before administration.

The dosage of the anti-MUC1 antibody-drug conjugate used in the present invention is preferably in the range of from 0.1 mg/kg to 50 mg/kg, more preferably in the range of from 1 mg/kg to 25 mg/kg, and even more preferably in the range of from 3 mg/kg to 10 mg/kg, per dose.

The dosing interval of the anti-MUC1 antibody-drug conjugate used in the present invention is preferably once a week (q1w), once every two weeks (g2w), once every three weeks (q3w), or once every four weeks (q4w), and is more preferably once every three weeks (q3w).

The anti-MUC1 antibody-drug conjugate used in the present invention can preferably be administered at a dose of 0.1 mg/kg to 50 mg/kg once a week to every four weeks, can more preferably be administered at a dose of 1 mg/kg to 25 mg/kg once every two to three weeks, and can even more preferably be administered at a dose of 3 mg/kg to 10 mg/kg once every three weeks.

## Examples

Hereinafter, the present invention will be specifically described with reference to examples. However, the present invention is not limited to these examples. These examples are not to be interpreted in a limited manner in any sense.

Example 1: Preparation of anti-MUC1 antibody-drug conjugate

In accordance with the method described in Example 1 of International Publication No. WO 2019/219891, an anti-TA-MUC1 antibody-drug conjugate was prepared, in which a drug-linker represented by the following formula:

[Chem. 8]

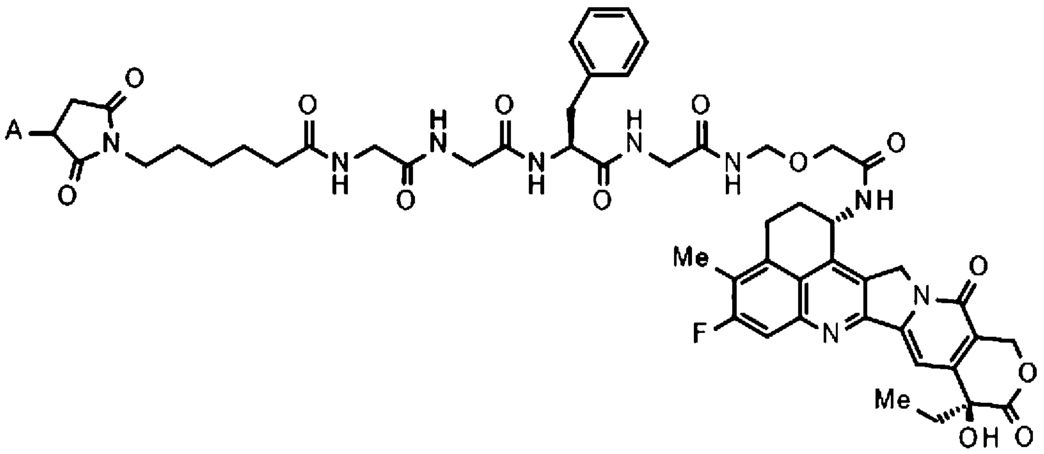

wherein A represents the connecting position to an antibody,
was conjugated to an anti-TA-MUC1 antibody via a thioether bond, using an anti-TA-MUC1 antibody (PM-N54Q) comprising a heavy chain consisting of the amino acid sequence represented by SEQ ID NO: 11 (Figure 3), and a light chain consisting of the amino acid sequence represented by SEQ ID NO: 12 (Figure 4) (in the present invention, this conjugate is referred to as TA-MUC1-DXd ADC). The average number of conjugated drug molecules per antibody in TA-MUC1-DXd ADC is in the range of from 7 to 8.

Reference Example 1: Preparation of sacituzumab govitecan

With reference to the method described in Example 12 of U.S. Patent No. 7999083, sacituzumab govitecan (TROP2-SN38 ADC) was prepared using an hRS7 antibody comprising a heavy chain consisting of the amino acid sequence represented by SEQ ID NO: 14, and a light chain consisting of the amino acid sequence represented by SEQ ID NO: 15.

Reference Example 2: Preparation of datopotamab deruxtecan

With reference to the method described in WO 2015/098099 and WO 2017/002776, datopotamab deruxtecan (TROP2-DXd ADC) was prepared, in which a drug-linker represented by the following formula:

[Chem. 9]

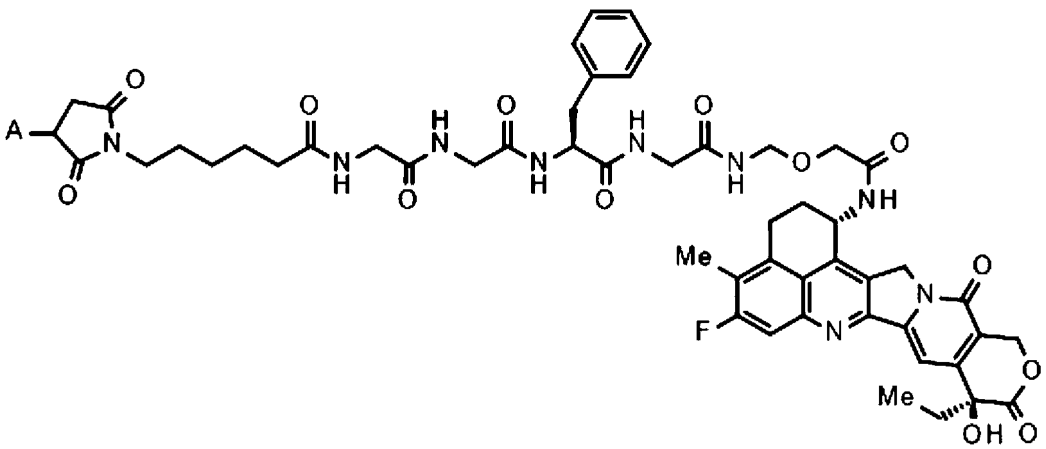

wherein A represents the connecting position to an antibody,
was conjugated to an anti-TROP2 antibody via a thioether bond, using an anti-TROP2 antibody comprising a heavy chain consisting of the amino acid sequence represented by SEQ ID NO: 26, and a light chain consisting of the amino acid sequence represented by SEQ ID NO: 27.

Reference Example 3: Preparation of trastuzumab deruxtecan

With reference to the method described in WO 2015/115091, trastuzumab deruxtecan (HER2-DXd ADC) was prepared, in which a drug-linker represented by the following formula:

[Chem. 10]

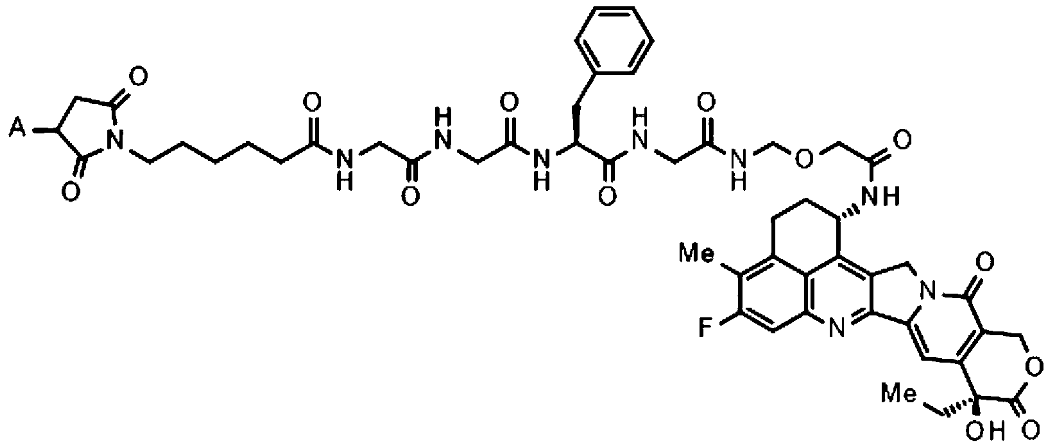

wherein A represents the connecting position to an antibody,
was conjugated to an anti-HER2 antibody via a thioether bond, using an anti-HER2 antibody comprising a heavy chain consisting of the amino acid sequence represented by SEQ ID NO: 36, and a light chain consisting of the amino acid sequence represented by SEQ ID NO: 37.

Example 2: Evaluation of antitumor activity of TA-MUC1-DXd ADC against bladder cancer patient-derived xenograft model

NIBIO-K071 was obtained as a bladder cancer patient-derived xenograft model (PDX model) from the National Institute of Biomedical Innovation. A frozen tumor section of NIBIO-K071 was thawed and subcutaneously transplanted to 4- to 6-week old female NSG mice (NOD.Cg-Prkdc<scid> Il2rg<tm1Wjl>/SzJ: purchased from Charles River Laboratories Japan, Inc.) using a trocar. Grown tumor was collected, cut into small pieces, and transplanted again to female NSG mice or female nude mice (CAnN.Cg-Foxn1nu/CrlCrlj: purchased from Charles River Laboratories Japan, Inc.) using a trocar, followed by extended growth. TA-MUC1-, Nectin-4-, and TROP2-positive findings were confirmed in the tumor of this model by immunohistochemical staining (IHC). An estimated tumor volume (ETV) was calculated as described below.

Estimated tumor volume (volume, $mm^3$) = Major axis (mm) $\times$ Minor axis $(mm)^2/2$

The female nude mice with transplanted NIBIO-K071 were grouped 25 days after transplantation when their average tumor volume became about 200 $mm^3$. Then, TA-MUC1-DXd ADC prepared in Example 1, Control IgG-DXd ADC, TROP2-SN38 ADC (prepared in Reference Example 1), or enfortumab vedotin (Nectin-4-MMAE ADC, which was prepared at 10 mg/mL from a freeze-dried product purchased from Gilead Sciences, Inc. using Otsuka distilled water (manufactured by Otsuka Pharmaceutical Factory, Inc.)) was diluted with ABS buffer (10 mM acetate buffer solution, pH 5.5, 5% sorbitol) and intravenously administered to the tail in a liquid volume of 10 mL/kg body weight of each mouse (Day 0). TA-MUC1-DXd ADC with DAR = 7.8 was used. The dosing schedules were set to QW $\times$ three doses (Days 0, 7, and 14) of Nectin-4-MMAE ADC (3 mg/kg), Q3W $\times$ two doses (Days 0 and 21) of TA-MUC1-DXd ADC and Control IgG-DXd ADC (3 or 10 mg/kg), and QW $\times$ two doses (Days 0 and 7) of TROP2-SN38 ADC (10 mg/kg). ABS buffer was administered to a Vehicle administration group. Six mice per group were used in all the groups. After initial administration, body weights and tumor sizes were measured twice a week, and estimated tumor volumes were calculated. The antigen dependence of the drug efficacy of TA-MUC1-DXd ADC was analyzed by the t test. The comparison of drug efficacy among the agents was analyzed by the parametric Dunnett's multiple comparison.

Figure 1 shows change in estimated tumor volume in the antitumor test described above. On Day 21, TA-MUC1-DXd ADC at 3 mg/kg and 10 mg/kg exhibited statistically significant antitumor activity as compared with the same doses of

Control IgG-DXd ADC as a negative control (Table 1) ($P < 0.0001$ in all the cases, the t test). TA-MUC1-DXd ADC in the 3 mg/kg and 10 mg/kg administration groups also exhibited statistically significant antitumor activity based on Nectin-4-MMAE ADC and TROP2-SN38 ADC (Table 1) ($P < 0.0001$ in all the cases, the parametric Dunnett's multiple comparison).

[Table 1]

| | | | **ETV ($mm^3$)** | | **Student's t-test (P value)** | **Dunnett's test (P value)** | |
|---|---|---|---|---|---|---|---|
| Group | Treatment | n | Mean | SE | vs. Control IgG-DXd ADC | **vs.** Group 6 | **vs.** Group 7 |
| 1 | Vehicle | 6 | 1289 | 62 | | | |
| 2 | Control IgG-DXd ADC, 3 mg/kg, Q3W | 6 | 1052 | 82 | | | |
| 3 | Control IgG-DXd ADC, 10 mg/kg, Q3W | 6 | 636 | 91 | | | |
| 4 | TA-MUC1-DXd ADC, 3 mg/kg, Q3W | 6 | 34 | **15** | 0.0001 (vs. 3mg/kg) | < 0.0001 | < 0.0001 |
| 5 | TA-MUC1-DXd ADC, 10 mg/kg, **Q3W** | 6 | 6 | 1 | < 0.0001 (vs. 10 mg/kg) | < 0.0001 | < 0.0001 |
| 6 | Nectin4-MMAE ADC, 3 mg/kg, QWx3 | 6 | 772 | 37 | | | |
| 7 | TROP2-SN38 ADC, 10 mg/kg, QWx2 | 6 | 383 | 73 | | | |

These results revealed that in the NIBIO-K071 model, TA-MUC1-DXd ADC exhibited significantly strong antitumor activity in an antigen-specific manner as compared with Nectin-4-MMAE ADC and TROP2-SN38 ADC.

TA-MUC1-DXd ADC was sequentially administered at 10 mg/kg on Days 21 and 28 to the Nectin-4-MMAE ADC administration group and the TROP2-SN38 ADC administration group, respectively, which did not reach tumor regression in this test, and evaluation and observation were further carried out for about 3 weeks. As a result, a strong regression effect was confirmed in both the groups after administration of TA-MUC1-DXd ADC. No reduction in body weight caused by sequential administration of the agents was found.

The results described above revealed that TA-MUC1-DXd ADC not only exhibited a significantly strong antitumor effect as compared with Nectin-4-MMAE ADC and TROP2-SN38 ADC but exhibited strong drug efficacy even on tumors found to have no regression by their administration.

Example 3: Antitumor evaluation of TA-MUC1-DXd ADC on model with transplanted breast cancer cell line HCC70

A human breast cancer cell line HCC70 was purchased from ATCC and reconstituted in an incubator set to 37°C and 5% $CO_2$ using RPMI 1640 medium (manufactured by Thermo Fisher Scientific Inc.) containing 10% fetal bovine serum. Then, the cells were passaged at a frequency of once to twice a week. The cells thus sufficiently increased in number were recovered from a flask using trypsin (manufactured by Thermo Fisher Scientific Inc.) and suspended at $1 \times 10^8$ cells/mL in Otsuka Normal Saline. The suspension was subcutaneously transplanted at 100 μL to each 5-week-old female nude mouse ($1 \times 10^7$ cells/100 μL/head). The mice were grouped 18 days after transplantation when their average tumor volume became about 165 $mm^3$.

Then, TA-MUC1-DXd ADC prepared in Example 1, Control IgG-DXd ADC, or TROP2-SN38 ADC (prepared at 10 mg/mL from a freeze-dried product purchased from Seagen Inc. using Otsuka Normal Saline (manufactured by Otsuka Pharmaceutical Factory, Inc.)) was diluted with ABS buffer and intravenously administered to the tail in a liquid volume of 10 mL/kg body weight of each mouse (Day 0). TA-MUC1-DXd ADC with DAR = 7.9 was used. The dosing schedules were set to Q3W × three doses (Days 0, 21, and 24) of TA-MUC1-DXd ADC (10 mg/kg), Q3W × one dose (Day 0) of Control IgG-DXd ADC (10 mg/kg), and QW × two doses (Days 0 and 7) of TROP2-SN38 ADC (10 mg/kg). ABS buffer was administered to a Vehicle administration group. Six mice per group were used in all the groups. After initial administration, body weights and tumor sizes were measured once to twice a week, and estimated tumor volumes were calculated. The significant difference test of drug efficacy of each agent based on TA-MUC1-DXd ADC was statistically analyzed by the parametric Dunnett's multiple comparison. TA-MUC1- and TROP2-positive findings were also confirmed in the tumor of this model by IHC staining.

Figure 2 shows change in estimated tumor volume in the HCC70 model. On Day 21, a strong regression effect was confirmed in the TA-MUC1-DXd ADC 10 mg/kg administration group, which exhibited statistically significant antitumor activity as compared with the Vehicle administration group, the Control IgG-DXd ADC administration group, and the TROP2-SN38 ADC administration group (Table 2).

[Table 2]

| | | | ETV ($mm^3$) | | Dunnett's test (P value) |
|---|---|---|---|---|---|
| Group | Treatment | n | Mean | SE | vs. TA-MUC1-DXd ADC |
| 1 | Vehicle, Q3W×1 | 6 | 655 | 104 | < 0.0001 |
| 2 | Control IgG-DXd ADC, 10 mg/kg, Q3W | 6 | 720 | 50 | < 0.0001 |
| 3 | TA-MUC1-DXd ADC, 10 mg/kg, Q3W | 6 | 19 | 4 | |
| 4 | TROP2-SN38 ADC, 10 mg/kg, QW×2 | 6 | 361 | 50 | 0.0028 |

These results revealed that in the HCC70 model, TA-MUC1-DXd ADC exhibited significantly strong antitumor activity in an antigen-specific manner as compared with TROP2-SN38 ADC.

TA-MUC1-DXd ADC was sequentially administered at 10 mg/kg on Days 21 and 42 to the TROP2-SN38 ADC administration group found to have no tumor regression in this test, and evaluation and observation were carried out up to Day 65. As a result, a strong regression effect was confirmed after administration of TA-MUC1-DXd ADC. No reduction in body weight caused by sequential administration of the agents was found.

The results described above revealed that TA-MUC1-DXd ADC not only exhibited a significantly strong antitumor effect as compared with TROP2-SN38 ADC in this model as well but exhibited strong drug efficacy even on tumors found to have no regression by administration of the latter.

Example 4: Evaluation of antitumor activity of TA-MUC1-DXd ADC and TROP2-DXd ADC against lung cancer patient-derived xenograft model NIBIO-NS1

NIBIO-NS1 was obtained as a lung cancer PDX model from the National Institute of Biomedical Innovation. A frozen tumor section of NIBIO-NS1 was thawed and subcutaneously transplanted to 5-week old female NSG mice (NOD.Cg-Prkdc<scid> Il2rg<tm1Wjl>/SzJ: purchased from Jackson Laboratory Japan, Inc.) using a trocar. Grown tumor was collected, cut into small pieces, and transplanted again to female nude mice (CAnN.Cg-Foxn1nu/CrlCrlj: purchased from Jackson Laboratory Japan, Inc.) using a trocar, followed by extended growth.

The female nude mice with transplanted NIBIO-NS1 were grouped 10 days after transplantation when their average tumor volume became about 157 $mm^3$. Then, TA-MUC1-DXd ADC prepared in Example 1 or TROP2-DXd ADC (prepared in Reference Example 2) was diluted with ABS buffer and intravenously administered to the tail in a liquid volume of 10 mL/kg body weight of each mouse (Day 0). Group configurations are shown in Table 3. ABS buffer was used in a Vehicle administration group, and TA-MUC1-DXd ADC with DAR = 7.9 and TROP2-DXd ADC with DAR = 4.0 were used. Six mice per group were used in groups 1 and 2, and 12 mice were used in group 3. Body weights and tumor sizes were measured once to twice a week, and estimated tumor volumes were calculated. The comparison of drug efficacy between agents was analyzed by the parametric Dunnett's multiple comparison. TA-MUC1- and TROP2-positive findings were confirmed in the tumor of this model by IHC staining.

Figure 6 shows change in estimated tumor volume in the antitumor test described above. On Day 11, a strong regression effect was confirmed in the TA-MUC1-DXd ADC 10 mg/kg administration group, which exhibited statistically significant antitumor activity as compared with the Vehicle administration group and the TROP2-DXd ADC 10 mg/kg administration group (Table 4).

[Table 3]

| **Group** | **Treatm ent** | **Dose** | **Admini stration** | **n** | **Gro up** | **Treatme nt** | **Dose** | **Admini stration** | **n** |
|---|---|---|---|---|---|---|---|---|---|
| 1 | Vehicle | | Day 0 | 6 | | | | | |
| 2 | TA-MUC1-DXd ADC | 10 mg/kg | Day 0 | 6 | | | | | |
| 3 | TROP2-DXd ADC | 10 mg/kg | Day 0 | 12 | 4<br>→ | TA-MUC1-DXd ADC | 10 mg/kg | Day 11 | 6 |

(continued)

| Group | Treatm ent | Dose | Admini stration | n | Gro up | Treatme nt | Dose | Admini stration | n |
|---|---|---|---|---|---|---|---|---|---|
| | | | | | 5 | TROP2-DXd ADC | 10 mg/ kg | Day 11 | 6 |

[Table 4]

| Group | | n | ETV ($mm^3$) Mean | SE | Dunnett's test (P value) vs. Group 2 |
|---|---|---|---|---|---|
| 1 | Vehicle | 6 | 780 | 79 | < 0.0001 |
| 2 | TA-MUC1-DXd ADC, 10 mg/kg | 6 | 51 | 3 | |
| 3 | TROP2-DXd ADC, 10 mg/kg | 12 | 453 | 42 | < 0.0001 |

These results revealed that the NIBIO-NS1 model has low sensitivity to TROP2-DXd ADC whereas TA-MUC1-DXd ADC in this model exhibited significantly strong antitumor activity as compared with TROP2-DXd ADC.

The tumor-bearing mice in the TROP2-DXd ADC administration group found to have no tumor regression in this test were regrouped such that each group involved six mice on the basis of their estimated tumor volumes on Day 11 when their average tumor volume became about 453 $mm^3$. TA-MUC1-DXd ADC was sequentially administered at 10 mg/kg to one of the groups, and evaluation and observation were carried out up to Day 31. TROP2-DXd ADC was continuously administered at 10 mg/kg to the other group, and evaluation and observation were carried out up to Day 21.

As a result, the tumor in the TROP2-DXd ADC administration group grew continuously, whereas a strong tumor regression effect was confirmed in the TROP2-DXd ADC → TA-MUC1-DXd ADC sequential administration group.

No reduction in body weight caused by sequential administration of the agents was found.

The results described above revealed that TA-MUC1-DXd ADC not only exhibited a significant antitumor effect in this model, which, albeit TROP2-positive, has low sensitivity to TROP2-DXd ADC, but also exhibited strong drug efficacy even on tumors found to have no regression after administration of TROP2-DXd ADC.

Example 5: Antitumor evaluation of TA-MUC1-DXd ADC, Control IgG-DXd ADC, and HER2-DXd ADC on model with transplanted breast cancer cell line HCC70

A human breast cancer cell line HCC70 was purchased from ATCC and reconstituted using RPMI 1640 medium (manufactured by Thermo Fisher Scientific Inc.) containing 10% fetal bovine serum. Then, the cells were cultured in an incubator set to 37°C and 5% $CO_2$, and passaged at a frequency of once to twice a week. The cells thus sufficiently increased in number were recovered from a flask using trypsin (manufactured by Thermo Fisher Scientific Inc.) and suspended at $1 \times 10^8$ cells/mL in D-PBS(-) (manufactured by FUJIFILM Wako Pure Chemical Corp.). The suspension was subcutaneously transplanted at 100 μL to each 5-week-old female nude mouse ($1 \times 10^7$ cells/100 μL/head). The mice were grouped 21 days after transplantation when the average tumor volume in the mice with the transplant became about 198 $mm^3$.

Then, TA-MUC1-DXd ADC prepared in Example 1, Control IgG-DXd ADC, or HER2-DXd ADC (prepared in Reference Example 3) was diluted with ABS buffer and intravenously administered to the tail in a liquid volume of 10 mL/kg body weight of each mouse (Day 0). Group configurations are shown in Table 5. ABS buffer was used in a Vehicle administration group, and TA-MUC1-DXd ADC with DAR = 7.9 and HER2-DXd ADC with DAR = 7.8 were used. Six mice per group were used in groups 1, 2, and 3, and 12 mice was used in group 4. Body weights and tumor sizes were measured once to twice a week, and estimated tumor volumes were calculated. The comparison of drug efficacy between agents was analyzed by the parametric Dunnett's multiple comparison. TA-MUC1 positivity and low HER2 expression were confirmed in the tumor of this model by IHC staining.

Figure 7 shows change in estimated tumor volume in the HCC70 model. On Day 21, a strong regression effect was confirmed in the TA-MUC1-DXd ADC 10 mg/kg administration group, which exhibited statistically significant antitumor activity as compared with the vehicle administration group, and the Control IgG-DXd ADC administration group and the HER2-DXd ADC administration group given the same doses thereas (Table 6).

[Table 5]

| Group | Treatm ent | Dose | Regime n Admini stration | n | | Gro up | Treatme nt | Dose | Regime n Admini stration | n |
|---|---|---|---|---|---|---|---|---|---|---|
| 1 | Vehicle | | Day 0 | 6 | | | | | | |
| 2 | Control IgG-DXd ADC | 10 mg/kg | Day 0 | 6 | | | | | | |
| 3 | TA-MUC1-DXd ADC | 10 mg/kg | Q3W x 3 Day 0, 21, 42 | 6 | | | | | | |
| 4 | HER2 DXd ADC | 10 mg/kg | Q3W x 1 Day 0 | 12 | → | 5 | TA-MUC1-DXd ADC | 10 mg/kg | Q3W x 3 Day 21, 42, 63 | 6 |
| | | | | | | 6 | HER2-DXd ADC | 10 mg/kg | Q3W x 1 Day 21 | 6 |

[Table 6]

| | | ETV ($mm^3$) | | Dunnett's test (P value) | |
|---|---|---|---|---|---|
| Group | n | Mean | SE | vs. Group 3 | vs. Group 4 |
| 1. Vehicle | 6 | 906 | 26 | < 0.0001 | < 0.0001 |
| 2. Control IgG-DXd ADC, 10 mg/kg | 6 | 636 | 32 | < 0.0001 | 0.0002 |
| 3. TA-MUC1-DXd ADC, 10 mg/kg | 6 | 43 | 5 | | < 0.0001 |
| 4. HER2-DXd-ADC, 10 mg/kg | 12 | 443 | 30 | < 0.0001 | |

These results revealed that the HCC70 model has low sensitivity to HER2-DXd ADC whereas TA-MUC1-DXd ADC in this model exhibited significantly strong antitumor activity as compared with HER2-DXd ADC.

The tumor-bearing mice in the HER2-DXd ADC administration group found to have no tumor regression in this test were regrouped such that each group involved six mice on the basis of their estimated tumor volumes on Day 21 when their average tumor volume became about 443 $mm^3$. TA-MUC1-DXd ADC was sequentially administered at 10 mg/kg (Q3W × 3) to one of the groups, and evaluation and observation were carried out up to Day 84. HER2-DXd ADC was continuously administered at 10 mg/kg to the other group, and evaluation and observation were carried out up to Day 42. As a result, the tumor in the HER2-DXd ADC administration group grew continuously, whereas a strong tumor regression effect was confirmed in the TA-MUC1-DXd ADC administration group. No reduction in body weight caused by sequential administration of the agents was found.

The results described above revealed that TA-MUC1-DXd ADC not only exhibited a significant antitumor effect in this model, which has low sensitivity to HER2-DXd ADC, but also exhibited strong drug efficacy even on tumors found to have no regression after administration of HER2-DXd ADC.

Free Text of Sequence Listing

SEQ ID NO: 1 - Amino acid sequence of CDRH1 of PM-N54Q and PankoMab-GEX(R)
SEQ ID NO: 2 - Amino acid sequence of CDRH2 of PM-N54Q
SEQ ID NO: 3 - Amino acid sequence of CDRH3 of PM-N54Q and PankoMab-GEX(R)
SEQ ID NO: 4 - Amino acid sequence of CDRL1 of PM-N54Q and PankoMab-GEX(R)
SEQ ID NO: 5 - Amino acid sequence of CDRL2 of PM-N54Q and PankoMab-GEX(R)
SEQ ID NO: 6 - Amino acid sequence of CDRL3 of PM-N54Q and PankoMab-GEX(R)
SEQ ID NO: 7 - Amino acid sequence of CDRH2 of PankoMab-GEX(R)
SEQ ID NO: 8 - Amino acid sequence of a heavy chain variable region of PM-N54Q
SEQ ID NO: 9 - Amino acid sequence of a light chain variable region of PM-N54Q and PankoMab-GEX(R)
SEQ ID NO: 10 - Amino acid sequence of a heavy chain variable region of PankoMab-GEX(R)
SEQ ID NO: 11 - Amino acid sequence of a heavy chain of PM-N54Q
SEQ ID NO: 12 - Amino acid sequence of a light chain of PM-N54Q and PankoMab-GEX(R)

SEQ ID NO: 13 - Amino acid sequence of a heavy chain of PankoMab-GEX (R)
SEQ ID NO: 14 - Amino acid sequence of a heavy chain of the hRS7 antibody
SEQ ID NO: 15 - Amino acid sequence of a light chain of the hRS7 antibody
SEQ ID NO: 16 - Amino acid sequence of an epitope
SEQ ID NO: 17 - Amino acid sequence of an epitope
SEQ ID NO: 18 - Amino acid sequence of CDRH1 of the anti-TROP2 antibody
SEQ ID NO: 19 - Amino acid sequence of CDRH2 of the anti-TROP2 antibody
SEQ ID NO: 20 - Amino acid sequence of CDRH3 of the anti-TROP2 antibody
SEQ ID NO: 21 - Amino acid sequence of CDRL1 of the anti-TROP2 antibody
SEQ ID NO: 22 - Amino acid sequence of CDRL2 of the anti-TROP2 antibody
SEQ ID NO: 23 - Amino acid sequence of CDRL3 of the anti-TROP2 antibody
SEQ ID NO: 24 - Amino acid sequence of a heavy chain variable region of the anti-TROP2 antibody
SEQ ID NO: 25 - Amino acid sequence of a light chain variable region of the anti-TROP2 antibody
SEQ ID NO: 26 - Amino acid sequence of a heavy chain of the anti-TROP2 antibody
SEQ ID NO: 27 - Amino acid sequence of a light chain of the anti-TROP2 antibody
SEQ ID NO: 28 - Amino acid sequence of CDRH1 of the anti-HER2 antibody
SEQ ID NO: 29 - Amino acid sequence of CDRH2 of the anti-HER2 antibody
SEQ ID NO: 30 - Amino acid sequence of CDRH3 of the anti-HER2 antibody
SEQ ID NO: 31 - Amino acid sequence of CDRL1 of the anti-HER2 antibody
SEQ ID NO: 32 - Amino acid sequence of CDRL2 of the anti-HER2 antibody
SEQ ID NO: 33 - Amino acid sequence of CDRL3 of the anti-HER2 antibody
SEQ ID NO: 34 - Amino acid sequence of a heavy chain variable region of the anti-HER2 antibody
SEQ ID NO: 35 - Amino acid sequence of a light chain variable region of the anti-HER2 antibody
SEQ ID NO: 36 - Amino acid sequence of a heavy chain of the anti-HER2 antibody
SEQ ID NO: 37 - Amino acid sequence of a light chain of the anti-HER2 antibody

## Claims

**1.** A treatment agent for a cancer having low sensitivity to an existing anti-cancer agent, comprising an anti-MUC1 antibody-drug conjugate as an active ingredient.

**2.** The treatment agent according to claim 1, wherein the anti-cancer agent is an alkylating agent, a platinum-containing drug, an antifolate, a pyridine metabolism inhibitor, a purine metabolism inhibitor, a ribonucleotide reductase inhibitor, a nucleotide analog, a topoisomerase inhibitor, a microtubule polymerization inhibitor, a microtubule depolymerization inhibitor, an antitumor antibiotic, an anti-hormonal agent, an EGFR inhibitor, a BTK inhibitor, a BCR-ABL inhibitor, an ALK inhibitor, a HER2 inhibitor, an angiogenesis inhibitor, a PARP inhibitor, an mTOR inhibitor, a membrane differentiation antigen targeting drug, or an antibody-drug conjugate.

**3.** The treatment agent according to claim 1, wherein the anti-cancer agent is an antibody-drug conjugate.

**4.** The treatment agent according to claim 3, wherein the antibody-drug conjugate is an anti-TROP2 antibody-drug conjugate, an anti-Nectin-4 antibody-drug conjugate, or an anti-HER2 antibody-drug conjugate.

**5.** The treatment agent according to claim 4, wherein the antibody-drug conjugate is enfortumab vedotin, sacituzumab govitecan, datopotamab deruxtecan, trastuzumab emtansine, or trastuzumab deruxtecan.

**6.** The treatment agent according to any one of claims 1 to 5, wherein the cancer expresses TA-MUC1.

**7.** The treatment agent according to claim 6, wherein the cancer is selected from the group consisting of ovarian cancer, breast cancer, pancreatic cancer, lung cancer, colon cancer, gastric cancer, liver cancer, kidney cancer, blood cancer, endometrial cancer, thyroid cancer, leukemia, seminoma, melanoma, carcinoma, teratoma, lymphoma, sarcoma, mesothelioma, neuroblastoma, glioma, rectal cancer, adrenal cancer, skin cancer, brain cancer, uterine cervix cancer, gut cancer, intestinal cancer, head-and-neck cancer, alimentary canal cancer, lymph node cancer, esophageal cancer, colorectal cancer, ear, nose and throat (ENT) cancer, prostate cancer, bladder cancer, uterine cancer, biliary tract cancer, and metastases thereof.

**8.** The treatment agent according to claim 6, wherein the cancer is breast cancer, lung cancer, or bladder cancer.

9. The treatment agent according to any one of claims 1 to 8, wherein the anti-MUC1 antibody is an anti-TA-MUC1 antibody.

10. The treatment agent according to any one of claims 1 to 9, wherein the anti-MUC1 antibody-drug conjugate is an anti-MUC1 antibody-drug conjugate, in which a drug-linker represented by the following formula:

[Chem. 1]

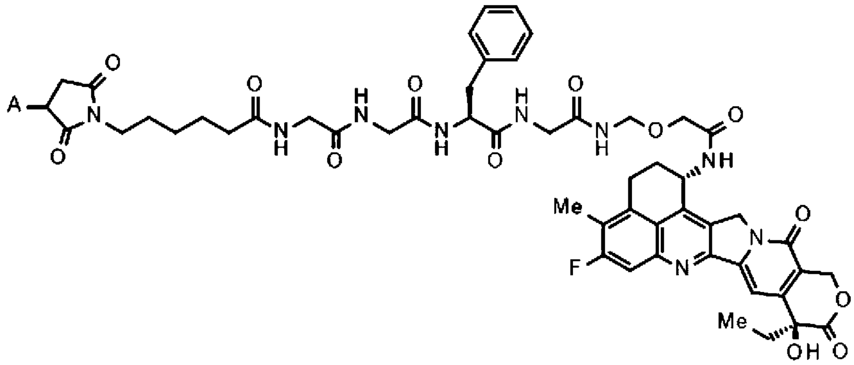

wherein A represents the connecting position to an anti-MUC1 antibody,
is conjugated to the anti-MUC1 antibody via a thioether bond.

11. The treatment agent according to any one of claims 1 to 10, wherein the anti-MUC1 antibody is an antibody comprising a heavy chain comprising CDRH1 consisting of the amino acid sequence represented by SEQ ID NO: 1, CDRH2 consisting of the amino acid sequence represented by SEQ ID NO: 2 or SEQ ID NO: 7, and CDRH3 consisting of the amino acid sequence represented by SEQ ID NO: 3, and a light chain comprising CDRL1 consisting of the amino acid sequence represented by SEQ ID NO: 4, CDRL2 consisting of the amino acid sequence represented by SEQ ID NO: 5, and CDRL3 consisting of the amino acid sequence represented by SEQ ID NO: 6.

12. The treatment agent according to any one of claims 1 to 10, wherein the anti-MUC1 antibody is an antibody comprising a heavy chain comprising CDRH1 consisting of the amino acid sequence represented by SEQ ID NO: 1, CDRH2 consisting of the amino acid sequence represented by SEQ ID NO: 2, and CDRH3 consisting of the amino acid sequence represented by SEQ ID NO: 3, and a light chain comprising CDRL1 consisting of the amino acid sequence represented by SEQ ID NO: 4, CDRL2 consisting of the amino acid sequence represented by SEQ ID NO: 5, and CDRL3 consisting of the amino acid sequence represented by SEQ ID NO: 6.

13. The treatment agent according to any one of claims 1 to 10, wherein the anti-MUC1 antibody is an antibody comprising a heavy chain comprising a heavy chain variable region consisting of the amino acid sequence represented by SEQ ID NO: 8 or SEQ ID NO: 10, and a light chain comprising a light chain variable region consisting of the amino acid sequence represented by SEQ ID NO: 9.

14. The treatment agent according to any one of claims 1 to 10, wherein the anti-MUC1 antibody is an antibody comprising a heavy chain comprising a heavy chain variable region consisting of the amino acid sequence represented by SEQ ID NO: 8, and a light chain comprising a light chain variable region consisting of the amino acid sequence represented by SEQ ID NO: 9.

15. The treatment agent according to any one of claims 1 to 10, wherein the anti-MUC1 antibody is an antibody comprising a heavy chain consisting of the amino acid sequence represented by SEQ ID NO: 11 or SEQ ID NO: 13, and a light chain consisting of the amino acid sequence represented by SEQ ID NO: 12.

16. The treatment agent according to any one of claims 1 to 10, wherein the anti-MUC1 antibody is an antibody comprising a heavy chain consisting of the amino acid sequence represented by SEQ ID NO: 11, and a light chain consisting of the amino acid sequence represented by SEQ ID NO: 12.

17. The treatment agent according to claim 15 or 16, wherein a lysine residue at the carboxyl terminus of the heavy chain

of the anti-MUC1 antibody is deleted.

**18.** The treatment agent according to any one of claims 1 to 17, wherein the average number of units of the drug-linker conjugated per antibody molecule in the anti-MUC1 antibody-drug conjugate is in the range of from 7 to 8.

**19.** The treatment agent according to any one of claims 1 to 17, wherein the average number of units of the drug-linker conjugated per antibody molecule in the anti-MUC1 antibody-drug conjugate is in the range of from 7.5 to 8.

**20.** A method for treating a cancer having low sensitivity to an existing anti-cancer agent, comprising administering an anti-MUC1 antibody-drug conjugate.

**21.** The treatment method according to claim 20, wherein the anti-cancer agent is an alkylating agent, a platinum-containing drug, an antifolate, a pyridine metabolism inhibitor, a purine metabolism inhibitor, a ribonucleotide reductase inhibitor, a nucleotide analog, a topoisomerase inhibitor, a microtubule polymerization inhibitor, a microtubule depolymerization inhibitor, an antitumor antibiotic, an anti-hormonal agent, an EGFR inhibitor, a BTK inhibitor, a BCR-ABL inhibitor, an ALK inhibitor, a HER2 inhibitor, an angiogenesis inhibitor, a PARP inhibitor, an mTOR inhibitor, a membrane differentiation antigen targeting drug, or an antibody-drug conjugate.

**22.** The treatment method according to claim 20, wherein the anti-cancer agent is an antibody-drug conjugate.

**23.** The treatment method according to claim 22, wherein the antibody-drug conjugate is an anti-TROP2 antibody-drug conjugate, an anti-Nectin-4 antibody-drug conjugate, or an anti-HER2 antibody-drug conjugate.

**24.** The treatment method according to claim 22, wherein the antibody-drug conjugate is enfortumab vedotin, sacituzumab govitecan, datopotamab deruxtecan, trastuzumab emtansine, or trastuzumab deruxtecan.

**25.** The treatment method according to any one of claims 20 to 24, wherein the cancer expresses TA-MUC1.

**26.** The treatment method according to claim 25, wherein the cancer is selected from the group consisting of ovarian cancer, breast cancer, pancreatic cancer, lung cancer, colon cancer, gastric cancer, liver cancer, kidney cancer, blood cancer, endometrial cancer, thyroid cancer, leukemia, seminoma, melanoma, carcinoma, teratoma, lymphoma, sarcoma, mesothelioma, neuroblastoma, glioma, rectal cancer, adrenal cancer, skin cancer, brain cancer, uterine cervix cancer, gut cancer, intestinal cancer, head-and-neck cancer, alimentary canal cancer, lymph node cancer, esophageal cancer, colorectal cancer, ear, nose and throat (ENT) cancer, prostate cancer, bladder cancer, uterine cancer, biliary tract cancer, and metastases thereof.

**27.** The treatment method according to claim 25, wherein the cancer is breast cancer, lung cancer, or bladder cancer.

**28.** The treatment method according to any one of claims 20 to 27, wherein the anti-MUC1 antibody is an anti-TA-MUC1 antibody.

**29.** The treatment method according to any one of claims 20 to 28, wherein the anti-MUC1 antibody-drug conjugate is an anti-MUC1 antibody-drug conjugate, in which a drug-linker represented by the following formula:

[Chem. 2]

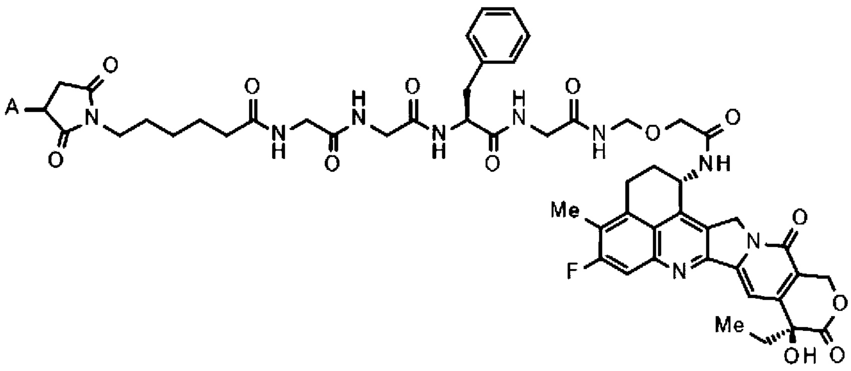

wherein A represents the connecting position to an anti-MUC1 antibody,
is conjugated to the anti-MUC1 antibody via a thioether bond.

**30.** The treatment method according to any one of claims 20 to 29, wherein the anti-MUC1 antibody is an antibody comprising a heavy chain comprising CDRH1 consisting of the amino acid sequence represented by SEQ ID NO: 1, CDRH2 consisting of the amino acid sequence represented by SEQ ID NO: 2 or SEQ ID NO: 7, and CDRH3 consisting of the amino acid sequence represented by SEQ ID NO: 3, and a light chain comprising CDRL1 consisting of the amino acid sequence represented by SEQ ID NO: 4, CDRL2 consisting of the amino acid sequence represented by SEQ ID NO: 5, and CDRL3 consisting of the amino acid sequence represented by SEQ ID NO: 6.

**31.** The treatment method according to any one of claims 20 to 29, wherein the anti-MUC1 antibody is an antibody comprising a heavy chain comprising CDRH1 consisting of the amino acid sequence represented by SEQ ID NO: 1, CDRH2 consisting of the amino acid sequence represented by SEQ ID NO: 2, and CDRH3 consisting of the amino acid sequence represented by SEQ ID NO: 3, and a light chain comprising CDRL1 consisting of the amino acid sequence represented by SEQ ID NO: 4, CDRL2 consisting of the amino acid sequence represented by SEQ ID NO: 5, and CDRL3 consisting of the amino acid sequence represented by SEQ ID NO: 6.

**32.** The treatment method according to any one of claims 20 to 29, wherein the anti-MUC1 antibody is an antibody comprising a heavy chain comprising a heavy chain variable region consisting of the amino acid sequence represented by SEQ ID NO: 8 or SEQ ID NO: 10, and a light chain comprising a light chain variable region consisting of the amino acid sequence represented by SEQ ID NO: 9.

**33.** The treatment method according to any one of claims 20 to 29, wherein the anti-MUC1 antibody is an antibody comprising a heavy chain comprising a heavy chain variable region consisting of the amino acid sequence represented by SEQ ID NO: 8, and a light chain comprising a light chain variable region consisting of the amino acid sequence represented by SEQ ID NO: 9.

**34.** The treatment method according to any one of claims 20 to 29, wherein the anti-MUC1 antibody is an antibody comprising a heavy chain consisting of the amino acid sequence represented by SEQ ID NO: 11 or SEQ ID NO: 13, and a light chain consisting of the amino acid sequence represented by SEQ ID NO: 12.

**35.** The treatment method according to any one of claims 20 to 29, wherein the anti-MUC1 antibody is an antibody comprising a heavy chain consisting of the amino acid sequence represented by SEQ ID NO: 11, and a light chain consisting of the amino acid sequence represented by SEQ ID NO: 12.

**36.** The treatment method according to claim 34 or 35, wherein a lysine residue at the carboxyl terminus of the heavy chain of the anti-MUC1 antibody is deleted.

**37.** The treatment method according to any one of claims 20 to 36, wherein the average number of units of the drug-linker conjugated per antibody molecule in the anti-MUC1 antibody-drug conjugate is in the range of from 7 to 8.

**38.** The treatment method according to any one of claims 20 to 36, wherein the average number of units of the drug-linker conjugated per antibody molecule in the anti-MUC1 antibody-drug conjugate is in the range of from 7.5 to 8.

[Figure 1]

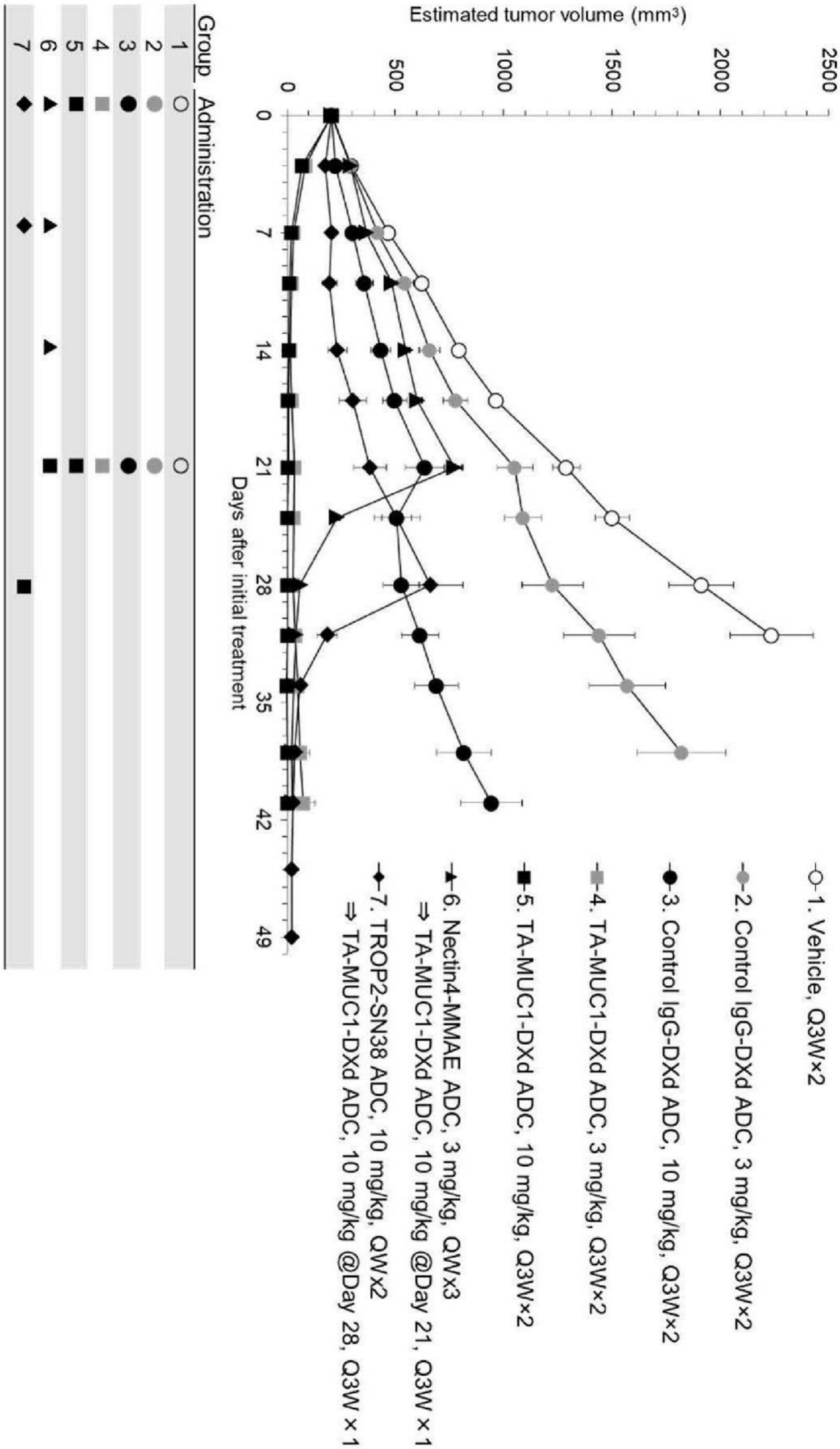
Estimated tumor volume (mm3)
0
500
1000
1500
2000
2500
0
7
14
21
28
35
42
49
Days after initial treatment
1. Vehicle, Q3W×2
2. Control IgG-DXd ADC, 3 mg/kg, Q3W×2
3. Control IgG-DXd ADC, 10 mg/kg, Q3W×2
4. TA-MUC1-DXd ADC, 3 mg/kg, Q3W×2
5. TA-MUC1-DXd ADC, 10 mg/kg, Q3W×2
6. Nectin4-MMAE ADC, 3 mg/kg, QWx3
⇒ TA-MUC1-DXd ADC, 10 mg/kg @Day 21, Q3W × 1
7. TROP2-SN38 ADC, 10 mg/kg, QW×2
⇒ TA-MUC1-DXd ADC, 10 mg/kg @Day 28, Q3W × 1
Group
Administration
1
2
3
4
5
6
7

[Figure 2]

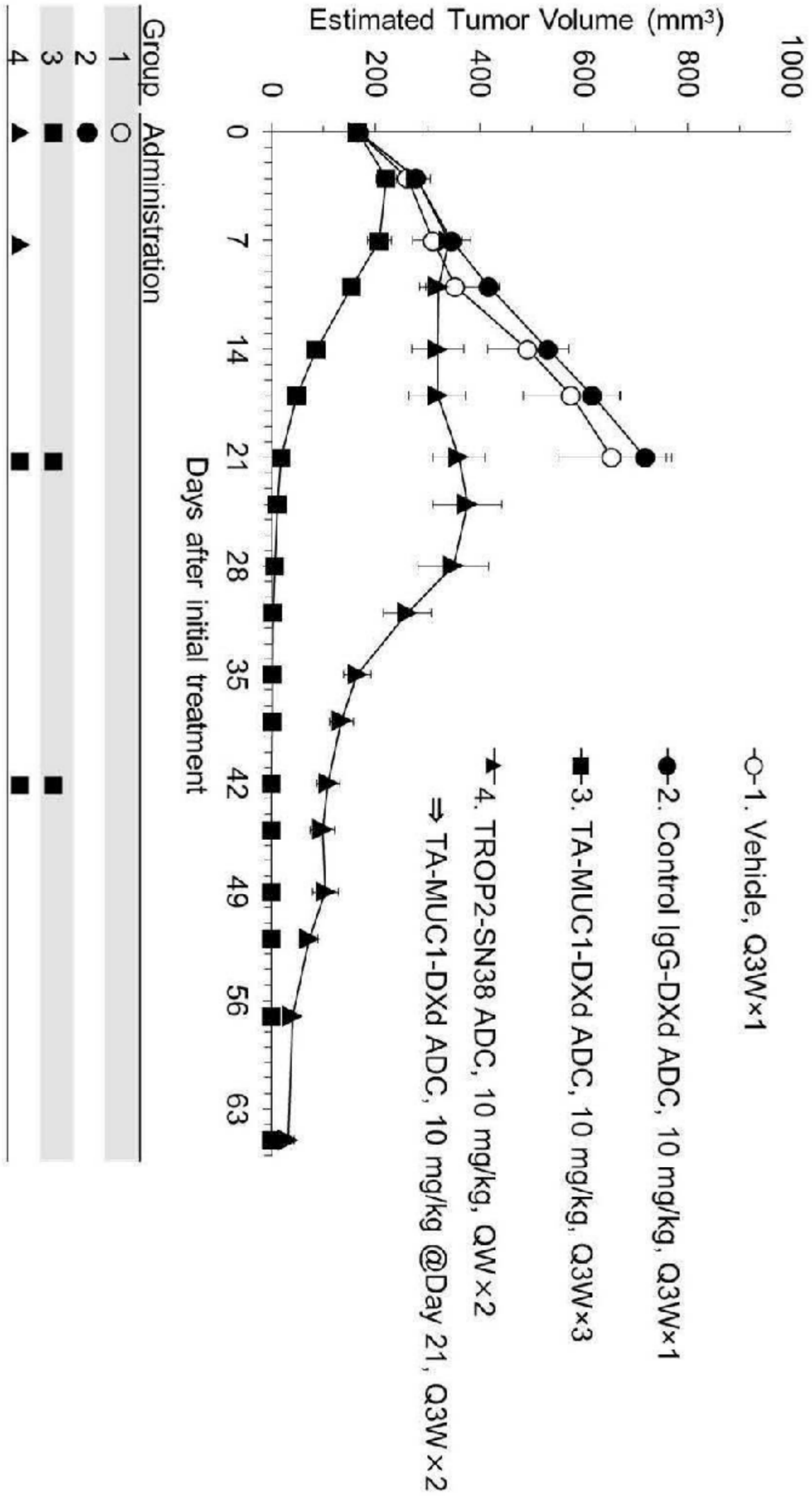
Estimated Tumor Volume (mm³)
0
200
400
600
800
1000
Days after initial treatment
0
7
14
21
28
35
42
49
56
63
1. Vehicle, Q3W×1
2. Control IgG-DXd ADC, 10 mg/kg, Q3W×1
3. TA-MUC1-DXd ADC, 10 mg/kg, Q3W×3
4. TROP2-SN38 ADC, 10 mg/kg, QW×2
⇒ TA-MUC1-DXd ADC, 10 mg/kg @Day 21, Q3W×2
Group
Administration
1
2
3
4

[Figure 3]

EVQLVESGGGLVQPGGSMRLSCVASGFPFSNYWMNWVRQAPGKGLEWVGEIRLKSN**Q**YTTH
YAESVKGRFTISRDDSKNSLYLQMNSLKTEDTAVYYCTRHYYFDYWGQGTLVTVSSASTKGPS
VFPLAPSSKSTSGGTAALGCLVKDYFPEPVTVSWNSGALTSGVHTFPAVLQSSGLYSLSSVVTVP
SSSLGTQTYICNVNHKPSNTKVDKKVEPKSCDKTHTCPPCPAPELLGGPSVFLFPPKPKDTLMIS
RTPEVTCVVVDVSHEDPEVKFNWYVDGVEVHNAKTKPREEQYNSTYRVVSVLTVLHQDWLN
GKEYKCKVSNKALPAPIEKTISKAKGQPREPQVYTLPPSRDELTKNQVSLTCLVKGFYPSDIAVE
WESNGQPENNYKTTPPVLDSDGSFFLYSKLTVDKSRWQQGNVFSCSVMHEGLHNHYTQKSLS
LSPGK

variable region: aa 1-117, constant region: aa 118-447

[Figure 4]

DIVMTQSPLSNPVTPGEPASISCRSSKSLLHSNGITYFFWYLQKPGQSPQLLIYQMSNLASGVPD
RFSGSGSGTDFTLRISRVEAEDVGVYYCAQNLELPPTFGQGTKVEIKRTVAAPSVFIFPPSDEQL
KSGTASVVCLLNNFYPREAKVQWKVDNALQSGNSQESVTEQDSKDSTYSLSSTLTLSKADYE
KHKVYACEVTHQGLSSPVTKSFNRGEC

variable region: aa 1-113, constant region: aa 114-219

[Figure 5]

EVQLVESGGGLVQPGGSMRLSCVASGFPFSNYWMNWVRQAPGKGLEWVGEIRLKSN**N**YTTH
YAESVKGRFTISRDDSKNSLYLQMNSLKTEDTAVYYCTRHYYFDYWGQGTLVTVSSASTKGPS
VFPLAPSSKSTSGGTAALGCLVKDYFPEPVTVSWNSGALTSGVHTFPAVLQSSGLYSLSSVVTVP
SSSLGTQTYICNVNHKPSNTKVDKKVEPKSCDKTHTCPPCPAPELLGGPSVFLFPPKPKDTLMIS
RTPEVTCVVVDVSHEDPEVKFNWYVDGVEVHNAKTKPREEQYNSTYRVVSVLTVLHQDWLN
GKEYKCKVSNKALPAPIEKTISKAKGQPREPQVYTLPPSRDELTKNQVSLTCLVKGFYPSDIAVE
WESNGQPENNYKTTPPVLDSDGSFFLYSKLTVDKSRWQQGNVFSCSVMHEGLHNHYTQKSLS
LSPGK

variable region: aa 1-117, constant region: aa 118-447

[Figure 6]

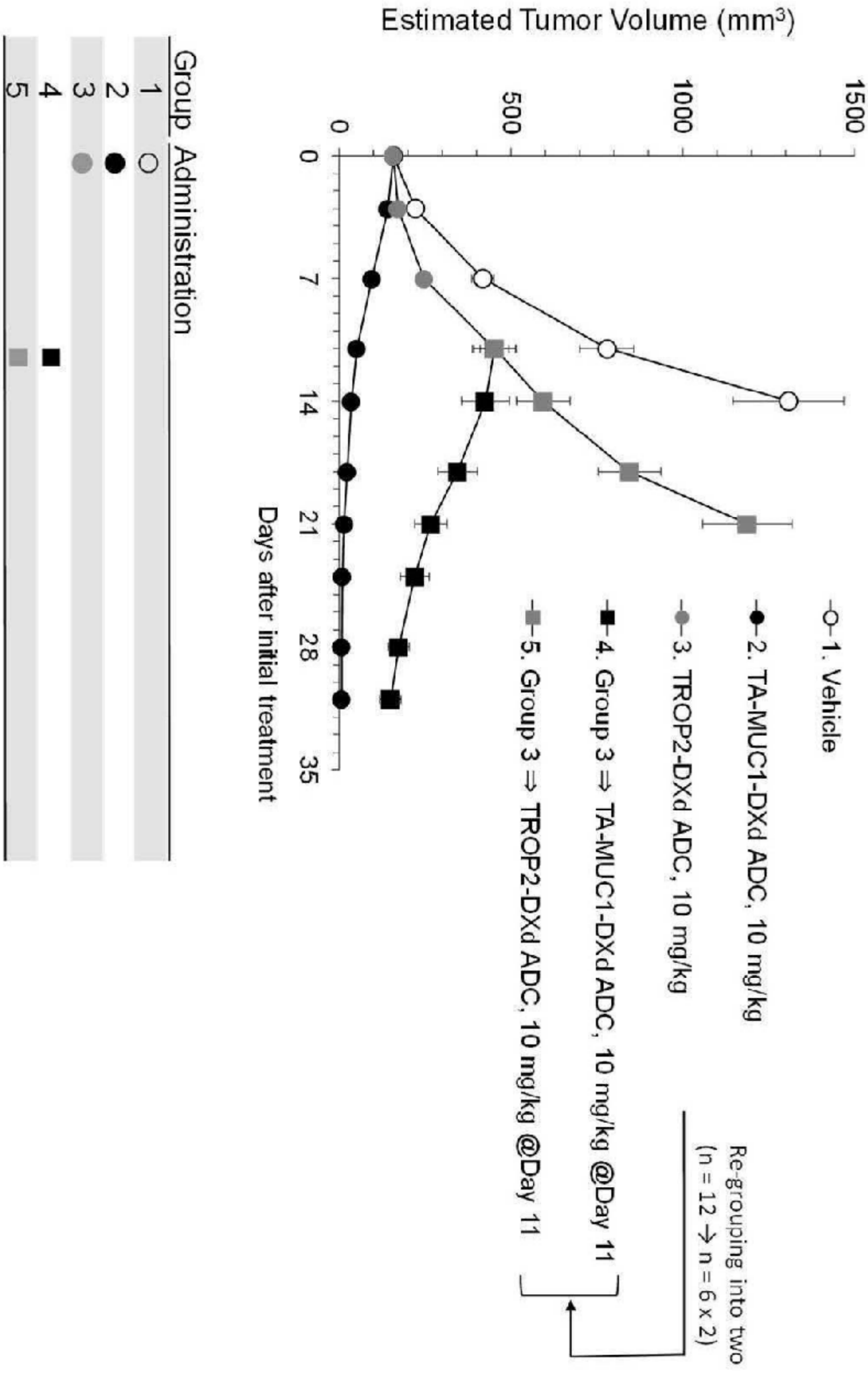
Estimated Tumor Volume (mm³)
0
500
1000
1500
0
7
14
21
28
35
Days after initial treatment
1. Vehicle
2. TA-MUC1-DXd ADC, 10 mg/kg
3. TROP2-DXd ADC, 10 mg/kg
4. Group 3 ⇒ TA-MUC1-DXd ADC, 10 mg/kg @Day 11
5. Group 3 ⇒ TROP2-DXd ADC, 10 mg/kg @Day 11
Re-grouping into two
(n = 12 → n = 6 x 2)
Group
Administration
1
2
3
4
5

[Figure 7]

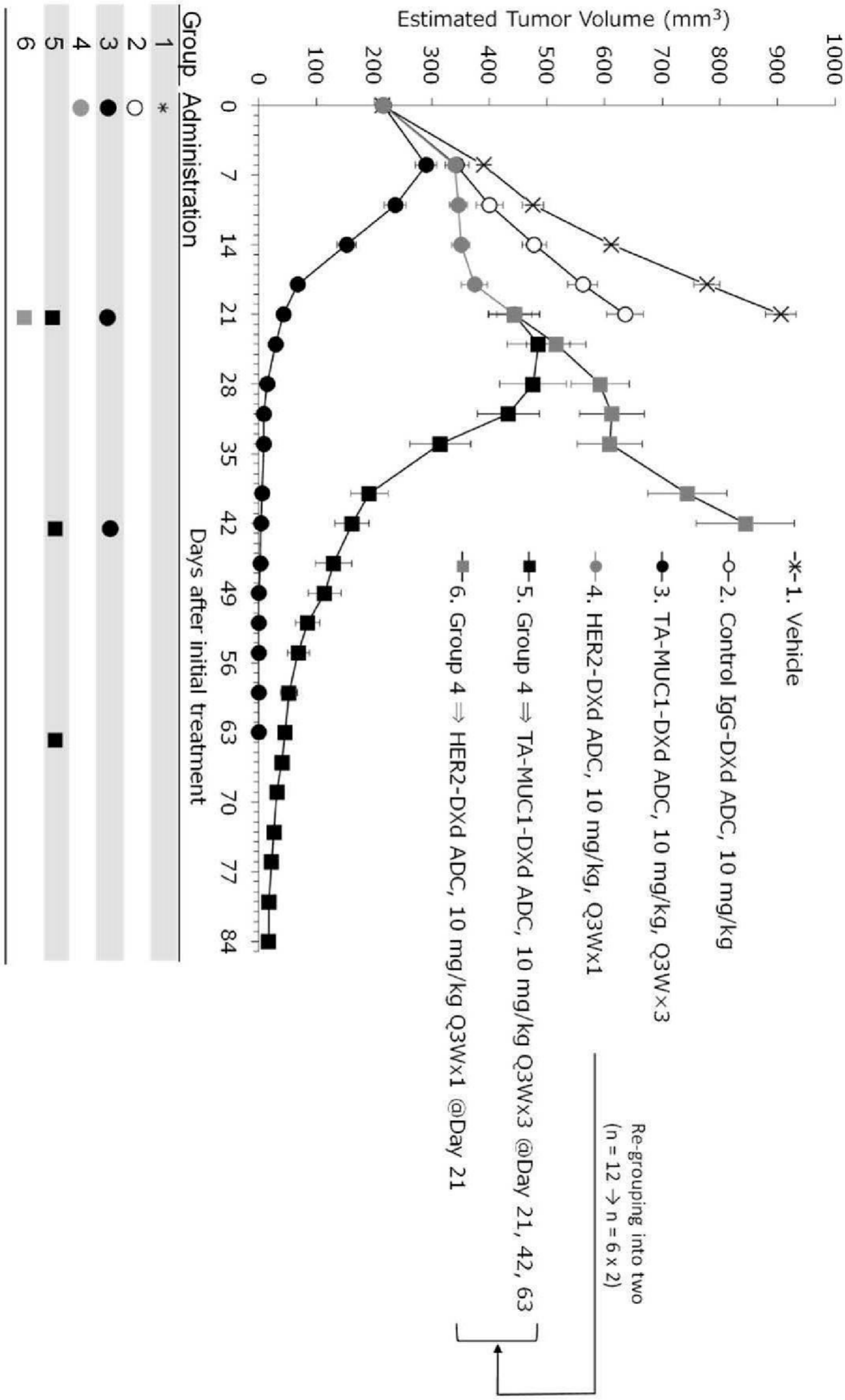
Estimated Tumor Volume (mm3)
0
100
200
300
400
500
600
700
800
900
1000
0
7
14
21
28
35
42
49
56
63
70
77
84
Days after initial treatment
1. Vehicle
2. Control IgG-DXd ADC, 10 mg/kg
3. TA-MUC1-DXd ADC, 10 mg/kg, Q3W×3
4. HER2-DXd ADC, 10 mg/kg, Q3Wx1
5. Group 4 ⇒ TA-MUC1-DXd ADC, 10 mg/kg Q3Wx3 @Day 21, 42, 63
6. Group 4 ⇒ HER2-DXd ADC, 10 mg/kg Q3Wx1 @Day 21
Re-grouping into two
(n = 12 → n = 6 x 2)
Group
Administration
1
2
3
4
5
6

**INTERNATIONAL SEARCH REPORT**

International application No. **PCT/JP2024/013882**

**A. CLASSIFICATION OF SUBJECT MATTER**

***A61K 47/68***(2017.01)i; ***A61K 31/4745***(2006.01)i; ***A61K 39/395***(2006.01)i; ***A61P 11/00***(2006.01)i; ***A61P 13/10***(2006.01)i; ***A61P 15/00***(2006.01)i; ***A61P 35/00***(2006.01)i; ***A61P 35/02***(2006.01)i; ***C07K 16/18***(2006.01)i; ***C07K 16/30***(2006.01)i; ***C12N 15/13***(2006.01)i

FI: A61K47/68 ZNA; A61K39/395 N; A61K39/395 T; A61P11/00; A61P15/00; A61P13/10; A61P35/00; A61K31/4745; C12N15/13; C07K16/18; A61P35/02; A61K39/395 L; C07K16/30

According to International Patent Classification (IPC) or to both national classification and IPC

**B. FIELDS SEARCHED**

Minimum documentation searched (classification system followed by classification symbols)

A61K47/68; A61K31/4745; A61K39/395; A61P11/00; A61P13/10; A61P15/00; A61P35/00; A61P35/02; C07K16/18; C07K16/30; C12N15/13

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
|---|---|---|
| X<br>Y | JP 2021-524852 A (DAIICHI SANKYO CO., LTD.) 16 September 2021 (2021-09-16)<br>claims, paragraphs [0026], [0069], [0100], examples 1, 9-10 | 1-16, 18-35, 37-38<br>1-38 |
| Y | NATH, Sritama et al., MUC1: a multifaceted oncoprotein with a key role in cancer progression, Trends in Molecular Medicine, 2014, vol. 20(6), pages 332-342<br>fig. 4, page 337, column of "Expression of TA-MUC1" | 1-38 |
| Y | QING, Liangliang et al., A prognosis marker MUC1 correlates with metabolism and drug resistance in bladder cancer: a bioinformatics research, BMC Urology, 2022, vol. 22:114, pages 1-13<br>abstract | 1-38 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **10 June 2024** | **25 June 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | <br><br>Telephone No. |

Form PCT/ISA/210 (second sheet) (July 2022)

INTERNATIONAL SEARCH REPORT

International application No.
**PCT/JP2024/013882**

**C. DOCUMENTS CONSIDERED TO BE RELEVANT**

| Category* | Citation of document, with indication, where appropriate, of the relevant passages | Relevant to claim No. |
|---|---|---|
| X | LI, Lan et al., Antitumor effect of a novel humanized MUC1 antibody-drug conjugate on triple-negative breast cancer, Heliyon, 03 April 2023, vol. 9, e15164, pages 1-9<br>abstract, fig. 6 | 1-8, 18-27, 37-38 |
| X | WU, Guang et al., A novel humanized MUC1 antibody-drug conjugate for the treatment of trastuzumab-resistant breast cancer, Acta Biochim. Biophys. Sin., 2021, vol. 53(12), pages 1625-1639<br>abstract, fig. 4, 7 | 1-8, 18-27, 37-38 |
| X | WU, Guang et al., Therapeutic effect of a MUC1-specific monoclonal antibody-drug conjugates against pancreatic cancer model, Cancer Cell International, 2022, vol. 22:417, pages 1-14<br>abstract, pages 1-2, column of background, first paragraph | 1-8, 18-27, 37-38 |
| A | BRASSARD, Julyanne et al., Antibody-Drug Conjugates Targeting Tumor-Specific Mucin Glycoepitopes, Front. Biosci. (Landmark Ed), 2022, vol. 27(11):301, pages 1-13<br>whole document | 1-38 |

Form PCT/ISA/210 (second sheet) (July 2022)

**INTERNATIONAL SEARCH REPORT**

International application No.
**PCT/JP2024/013882**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/JP2024/013882**

| Patent document cited in search report | Publication date (day/month/year) | Patent family member(s) | Publication date (day/month/year) |
|---|---|---|---|
| JP 2021-524852 A | 16 September 2021 | US 2021/0187118 A1<br>claims, paragraphs [0031], [0080], [0362], examples 1, 9-10<br>EP 3794041 A1<br>KR 10-2021-0010565 A<br>CN 112351999 A | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2010093395 A
- US 7999083 B
- WO 2015115091 A
- WO 2019219891 A
- WO 9007861 A
- US 5821337 A
- WO 2011012309 A
- WO 2019219889 A
- US 7097840 B
- WO 2014057687 A
- WO 2017083582 A
- WO 2021247798 A
- WO 2015155998 A
- WO 2015098099 A
- WO 2017002776 A


### Non-patent literature cited in the description


- **NATH S** ; **MUKHERJEE P.** *Trends Mol Med.*, 2014, vol. 20 (6), 332-42
- **QING L** ; **LI Q** ; **YANG Y et al.** *BMC Urol.*, 2022, vol. 22 (1), 114
- **GENDLER SJ** ; **LANCASTER CA** ; **TAYLOR-PAPADIMITRIOU J et al.** *J Biol Chem.*, 1990, vol. 265 (25), 15286-93
- **HANISCH FG** ; **MULLER S.** *Glycobiology.*, 2000, vol. 10 (5), 439-49
- **KOHLER** ; **MILSTEIN**. *Nature*, 1975, vol. 256, 495-497
- Monoclonal Antibodies. Plenum Press, 1980, 365-367
- *Proc. Natl. Acad. Sci. USA*, 1984, vol. 81, 6851-6855
- *Nature*, 1986, vol. 321, 522-525
- **TOMIZUKA, K. et al.** *Nature Genetics*, 1997, vol. 16, 133-143
- **KUROIWA, Y.** *Nucl. Acids Res.*, 1998, vol. 26, 3447-3448
- **YOSHIDA, H.** Animal Cell Technology: Basic and Applied Aspects. Kluwer Academic Publishers, 1999, vol. 10, 69-73
- **TOMIZUKA, K.** *Proc. Natl. Acad. Sci. USA*, 2000, vol. 97, 722-727
- **CARMEN, S.** *Briefings in Functional Genomics and Proteomics*, 2002, vol. 1 (2), 189-203
- **SIRIWARDENA, D.** *Ophthalmology*, 2002, vol. 109 (3), 427-431
- *Journal of Chromatography A*, 1995, vol. 705, 129-134
- *Analytical Biochemistry*, 2007, vol. 360, 75-83
- **HERMANSON, G. T**. Bioconjugate Techniques. Academic Press, 1996, 56-136, 456-493